# FASCICULE DE BREVET EUROPEEN

(11) **EP 3 996 613 B1**
(45) Date de publication et mention de la délivrance du brevet: **16.04.2025**
(21) Numéro de dépôt: 20757529.1
(22) Date de dépôt: 07.07.2020
(51) Int. Cl.: A61B 17/70, A61B 17/88, A61B 17/00

(54) **IMPLANT VERTÉBRAL INTERÉPINEUX ET ANCILLAIRE DE POSE ASSOCIÉ**
INTERSPINALES IMPLANTAT UND ZUGEHÖRIGES IMPLANTATIONSHILFSMITTEL
INTERSPINOUS IMPLANT AND ASSOCIATED IMPLANTATION ANCILLARY

(30) Priorité: 08.07.2019 FR 1907608; 08.07.2019 FR 1907609
(43) Date de publication de la demande: 18.05.2022
(73) Titulaire: Innospina Sarl, 2822 Courroux (CH)
(72) Inventeur: HANNEMA, Gwenael Loïc, 8051 ZURICH (CH); SAMANI, Jacques, 97133 SAINT BARTHELEMY (FR); BEUCHAT, Dominique Constant, 2843 CHATILLON (CH)
(74) Mandataire: Germain Maureau
(86) Numéro de dépôt international: PCT/EP2020/069143
(87) Numéro de publication internationale: WO 2021/005070

(56) Documents cités:
- EP-A1- 3 294 166
- US-A1- 2013 190 820
- US-A1- 2016 242 824
- US-B2- 8 672 977
- US-B2- 9 101 409

## Description

L'invention se rapporte au domaine technique des implants vertébraux interépineux, et également des ancillaires de pose de tels implants vertébraux interépineux.

Les implants vertébraux interépineux sont des implants destinés à être situés entre les apophyses épineuses de deux vertèbres adjacentes. De tels implants permettent la distraction de deux apophyses épineuses adjacentes l'une par rapport à l'autre, c'est-à-dire l'obtention d'un écartement ou d'un espacement entre elles.

L'invention trouve notamment son application dans le traitement des douleurs lombaires par exemple.

Un implant vertébral interépineux connu de l'état de la technique, notamment du document US 2012/0265246, comporte un corps d'implant comprenant une partie centrale, destinée à s'étendre entre deux apophyses épineuses adjacentes, une partie avant, présentant une forme profilée adaptée pour écarter les apophyses épineuses des vertèbres progressivement et se frayer un chemin jusqu'entre les deux vertèbres et un canal de guidage se présentant sous la forme d'un trou de guidage rectiligne ménagé à travers le corps d'implant, débouchant uniquement sur les extrémités avant et arrière du corps d'implant, et destiné à recevoir une aiguille de guidage.

Une intervention chirurgicale est nécessaire pour mettre en place l'implant entre deux vertèbres. Cette intervention peut être percutanée ou ouverte et est réalisée selon un abord latéral du patient dans le cadre du document US 2012/0265246, bien qu'un abord dorsal soit également envisageable mais avec d'autres modèles d'implants vertébraux interépineux. Dans tous les cas de figure, une telle intervention nécessite un matériel conséquent pour repérer l'emplacement exact de l'implantation et se frayer un chemin jusqu'audit emplacement.

En effet, d'après le document US 2012/0265246, on utilise notamment une aiguille de guidage prévue pour s'engager dans le trou de guidage rectiligne ménagé dans l'implant pour le guider lors de son implantation, et le long de laquelle deux sortes d'outils permettent de dégager les tissus bloquant l'accès à l'espace intervertébral et écartent partiellement ou provisoirement les processus épineux pour une meilleure implantation. Le premier outil présente une tête en forme de vis ou de forme profilée lisse et glisse le long de l'aiguille de guidage. Le premier outil est configuré pour écarter provisoirement les processus épineux tout en sectionnant le ligament interépineux.

Une fois ce premier dégagement opéré, un deuxième outil est inséré le long de l'aiguille de guidage, le deuxième outil présente la même forme globale que le premier outil mais est de dimension plus large, de sorte à dégager plus de tissus et à préparer plus conséquemment l'emplacement de l'implantation. Il est décrit que plusieurs outils peuvent être utilisés successivement, jusqu'à ce que l'emplacement soit complètement dégagé ou qu'il soit de dimension suffisante pour recevoir l'implant. L'implant est alors placé dans l'emplacement dégagé en glissant le long de l'aiguille de guidage.

Cette méthode connue et décrite est contraignante, car elle nécessite plusieurs opérations afin de placer l'implant, elle requiert un nombre conséquent d'outils avant implantation. En outre, la chirurgie par abord latéral est souvent pratiquée sous anesthésie générale ce qui implique une procédure longue, complexe, avec une ouverture large dans le patient. Par ailleurs, le risque de malposition est important du fait de la distance entre la peau et la zone d'insertion.

L'état de la technique peut également être illustré par les enseignements du document US8672977 qui divulgue un implant vertébral interépineux comportant un corps d'implant de type articulé, pourvu d'une pluralité de segments successifs et interconnectés, pour être d'abord dans une configuration linéaire (pour son insertion) puis dans une configuration incurvée (une fois implanté entre deux apophyses épineuses adjacentes. Ainsi, au repos, ce corps d'implant est en configuration linéaire et il est traversé par un élément flexible de contraction qui passe à travers un canal qui se présente sous la forme de trous alignés ménagés dans les segments. Au repos, ces trous sont alignés et le canal est linéaire pour une introduction de l'élément flexible de contraction, qui va servir à tirer sur un segment à l'avant du corps d'implant, afin de pouvoir le faire passer de la configuration linéaire vers la configuration incurvée.

L'implant décrit dans ce document US8672977 présente cependant l'inconvénient d'être formé d'un corps articulé, qui passe de la configuration linéaire vers la configuration incurvée, ce qui lui procure une tenue mécanique réduite, et nécessairement moins fiable pour assurer une fonction de distraction de deux apophyses épineuses adjacentes. Par ailleurs, en passant de la configuration linéaire vers la configuration incurvée lors de son implantation, il existe des risques de coincement de matière entre les segments du corps d'implant qui sont d'abord espacés puis qui sont en contact, ce qui peut provoquer des pincements et des endommagements chez le patient, et également gêner l'implatation.

L'invention vise entre autre à se passer d'un corps d'implant articulé, en retenant l'emploi d'un corps d'implant non articulé plus fiable et moins traumatique lors de son implantation.

Il est aussi connu du document US9101409 d'employer un implant vertébral interépineux comportant un corps d'implant de type ogive de révolution, comprenant une partie arrière, une partie centrale affinée et une partie avant fuselée, où un trou axial est ménagé au centre du corps d'implant pour le traverser sur toute sa longueur ; ce trou axial servant à faire coulisser le corps d'implant le long d'une tige de guidage rectiligne selon une voie d'abord latéral au moyen d'un tournevis pousseur qui a une tête hexagonale emmanchée dans un orifice hexagonal prévu à l'arrière du trou axial, de sorte que l'implant est mis en place par un mouvement combinant une poussée latérale et une rotation, ce qui est équivalent à une implantation par « vissage ». Il est à noter que le corps d'implant présente également des rainures ménagées selon la direction longitudinale sur la moitié de la partie avant, l'intégralité de la partie centrale et la moitié de la partie arrière ; de telles rainures étant très fines et prévues pour donner de la flexibilité à l'implant, afin de l'autoriser à se contracter lors de son implantation et ainsi réduire le stress sur le patient.

Comme précédemment évoqué, de par son implantation selon une voie d'abord latéral, l'implant du document US9101409 présente des inconvénients en matière de complexité de la procédure d'implantation, majoritairement à l'aveugle et donc peu fiable, et aussi en matière de traumatisme pour le patient, malgré les rainures qui peuvent être insuffisantes pour réduire un tel stress. Par ailleurs, son implantation par « vissage » induit des torsions sur les tissus et sur les apophyses épineuses, qui peuvent être sources de traumatismes.

Il est aussi connu du document US2012/0226313 d'employer un implant vertébral interépineux comportant un corps d'implant de forme arquée, munie d'une fente latérale destinée à recevoir deux doigts de blocage, et munie d'une vis à l'arrière propre à appliquer un effort de serrage sur l'un des deux doigts de blocage ; les deux doigts de blocage étant prévus pour enchâsser à droite et à gauche les deux apophyses épineuses adjacentes, tandis que le corps d'implant est logé entre ces deux apophyses épineuses. Un tel implant présente tout de même une fiabilité mécanique limitée avec un implant composé de trois éléments (un corps d'implant et deux doigts de blocage ) qui doivent être assemblés entre eux. Cet implant coopère avec un ancillaire de pose comprenant une plateforme centré sur un poinçon et qui sert de support aux autres éléments, avec un inconvénient majeur qui est l'instabilité d'une telle plateforme durant toutes les opérations, sans compter son aspect traumatisant car faisant appel à plusieurs incisions, une pour passer le corps d'implant et une à deux autres pour passer les deux doigts de blocage. Les autres éléments sont une broche incurvée de forme arquée à 90 degrés qui est solidaire d'un bras de levier articulé à pivot sur la plateforme, et et un tube de guidage incurvé qui définit un canal incurvé et qui est solidaire d'un bras de levier monté pivotant sur la plateforme. Lors de l'implantation, une fois la plateforme en place, et malgré son défaut de stabilité, la broche incurvée pivote sur la plateforme pour venir positionner sa pointe entre les deux apophyses épineuses, puis le tube de guidage incurvé pivote aussi sur la plateforme en étant guidé par la broche incurvée qui se trouve dans le canal incurvé. Ensuite, la broche incurvée est retirée, et il est alors possible de pousser le corps d'implant à l'intérieur du canal incurvé du tube de guidage incurvé, jusqu'à se positionner entre les deux apophyses. Enfin, les deux doigts de guidage vont venir coulisser dans deux autres tubes incurvés également montés à pivot sur la plateforme. La méthode implantation décrite dans ce document US2012/0226313 présente plusieurs inconvénients, dont le défaut de stabilité de la plateforme précédemment évoqué, ainsi que les insertions traumatisantes de la broche incurvée et des trois tubes incurvés du fait de leurs formes arquées à 90 degrés qui leurs confèrent des dimensions telles que leurs implantations sera nécessairement traumatisante car leurs insertions appelent de longs débattements.

L'invention vise à remédier en tout ou partie aux inconvénients précités en proposant un implant vertébral interépineux adapté à une méthode d'implantation plus simple d'abord majoritairement dorsal et minoritairement latéral, c'est-à-dire qui fait appel à une approche dorsale mais excentrée légèrement de l'axe dorsal (ou axe de la colonne vertébrale). En effet, l'ouverture du patient est réalisée sur le dos sur une portion minimale nécessaire déterminée en fonction de la zone d'insertion de l'implant et en fonction de la taille de l'implant et de la taille de l'outil de pose, ce qui permet une implantation fiable et précise de l'implant sous anesthésie locale, et donc sans intervention chirurgicale lourde avec anesthésie générale, minimisant ainsi les risques de complications postopératoires.

L'invention propose également un ancillaire de pose spécialement adapté pour un implant vertébral interépineux selon l'invention, et qui est adapté à une méthode d'implantation entre les apophyses épineuses de deux vertèbres adjacentes (et non entre les plateaux vertébraux) plus simple d'abord majoritairement dorsal.

A cet effet, l'invention a pour objet un implant vertébral interépineux comportant un corps d'implant s'étendant selon un axe longitudinal et comprenant successivement le long dudit axe longitudinal :
- une partie arrière présentant une extrémité arrière,
- une partie centrale conformée pour s'étendre entre deux apophyses épineuses de deux vertèbres adjacentes, ladite partie centrale prolongeant la partie arrière, et
- une partie avant prolongeant la partie centrale, de manière opposée à la partie arrière, en s'effilant jusqu'à une extrémité avant de forme fuselée,

dans lequel ledit corps d'implant présente :
   - une face antérieure destinée à être orientée vers les vertèbres,
   - un canal de guidage ménagé dans le corps d'implant de son extrémité arrière jusqu'à son extrémité avant, ledit canal de guidage étant conformé pour recevoir au moins une portion d'une broche d'implantation d'un ancillaire de pose ;
ledit implant vertébral interépineux étant remarquable en ce que le canal de guidage s'étend depuis l'extrémité arrière jusqu'à l'extrémité avant suivant une direction curviligne, ledit canal de guidage étant ménagé dans la face antérieure de manière à ce que ledit canal de guidage forme une rainure antérieure de forme curviligne et débouchant extérieurement dans ladite face antérieure du corps d'implant.

Ainsi, un tel implant selon l'invention permet, grâce à une telle rainure antérieure, de recevoir une broche d'implantation incurvée de manière prédéterminée. Par conséquent, un tel implant selon l'invention autorise une intervention selon une voie d'abord majoritairement dorsale, ce qui est difficilement envisageable, voire impossible, avec un trou de guidage rectiligne de l'état de la technique. En effet, sa forme curviligne permet une insertion intuitive et progressive entre les vertèbres en faisant appel à une ouverture dorsale, légèrement excentrée de l'axe dorsal, favorisant ainsi une implantation directe dans la zone d'implantation déterminée.

Dans la présente invention, les termes suivants sont définis :
- par « avant », on entend la partie du corps d'implant qui est destiné à pénétrer en premier dans le corps du patient,
- par « arrière », on entend la partie du corps d'implant qui est destiné à pénétrer en dernier dans le corps du patient,
- par « forme fuselée », on entend la forme de l'extrémité avant qui va en s'amincissant selon la direction longitudinale selon un sens de l'arrière vers l'avant,
- par « direction curviligne », on entend que la direction du canal traversant suit une courbe, et non une droite, et par exemple une courbe en arc de cercle de rayon de courbure donnée ;
- par « face antérieure », on se réfère à l'anatomie du patient pour indiquer une face tournée vers les vertèbres ;
- par « face postérieure », on se réfère à l'anatomie du patient pour indiquer une face tournée à l'opposée des vertèbres, du côté de la peau du dos ;
- par « face inférieure », on se réfère à l'anatomie du patient pour indiquer une face tournée vers une apophyse épineuse de la vertèbre sous-jacente (vertèbre du dessous) ;
- par « face supérieure », on se réfère à l'anatomie du patient pour indiquer une face tournée vers une apophyse épineuse de la vertèbre sus-jacente (vertèbre du dessus). L'implant selon l'invention peut comporter une ou plusieurs des caractéristiques suivantes prises seules ou en combinaison.

Selon une caractéristique, la rainure antérieure est ménagée de manière continue (autrement dit sans interruption ni coupure) dans la face antérieure du corps d'implant depuis l'extrémité arrière jusqu'à l'extrémité avant, de manière à déboucher continuellement dans la face antérieure.

Selon une autre caractéristique, le corps d'implant est non articulé, de sorte que, dans une position de repos (avant implantation) comme dans une position implantée (une fois implanté entre les deux apophyses épineuses), la rainure antérieure est de forme curviligne selon une même courbure.

Selon une autre caractéristique, la rainure antérieure est de forme convexe selon un rayon de courbure donné, avec un centre de courbure situé en regard d'une face postérieure du corps d'implant, opposée à la face antérieure.

La forme convexe, qui correspond à une direction curviligne en arc de cercle, est avantageuse car elle permet à l'implant de passer d'une orientation verticale à une orientation horizontale selon une trajectoire de guidage souple et sans heurt de l'implant tout le long de son insertion.

Selon une possibilité, la face antérieure est de forme convexe selon le rayon de courbure.

Ainsi, le guidage de l'implant peut se faire aussi en se guidant sur cette face antérieure également curviligne.

Selon une autre possibilité, la rainure antérieure est délimitée intérieurement par une paroi de fond décalée en profondeur par rapport à la face antérieure, ladite paroi de fond étant de forme convexe selon un rayon de courbure donné.

Ainsi, le guidage de l'implant peut se faire aussi en se guidant sur cette paroi de fond également curviligne.

Selon une variante, la paroi de fond et la face antérieure ont le même centre de courbure, et le rayon de courbure de la paroi de fond est inférieur au rayon de courbure de la face antérieure.

Selon une autre possibilité, la rainure antérieure est délimitée extérieurement par des lèvres de retenue de forme convexe selon le rayon de courbure, lesdites lèvres de retenue étant disposées en regard de la paroi de fond et s'étendant l'une en face de l'autre pour délimiter un espace rétréci comparativement à l'intérieur de la rainure antérieure.

Ainsi, la rainure antérieure présente une section en « C », avec des lèvres de retenue aux terminaisons du « C » qui vont procurer une fonction de retenue de l'implant, autrement de maintien de l'implant sur la broche d'implantation sans risque de sortir de la broche d'implantation pendant l'insertion.

Dans une réalisation particulière, le rayon de courbure est compris entre 20 et 300 millimètres.

Un avantage procuré par de tels rayons de courbure est de faciliter l'intervention chirurgicale selon une voie d'abord majoritairement dorsale (avec une ouverture dorsale légèrement excentrée par rapport à l'axe dorsal), et de limiter la taille de l'incision.

Dans un mode de réalisation particulier, la partie arrière présente des moyens de pivotement selon un axe transversal s'étendant transversalement par rapport à l'axe longitudinal pour permettre un pivotement du corps d'implant relativement à des moyens de pivotement complémentaires prévus sur un porte-implant de l'ancillaire de pose.

Ainsi, ces moyens de pivotement vont permettre à l'implant de pivoter aisément lors de son insertion, son insertion se faisant suivant une trajectoire d'insertion ayant au moins une composante curviligne, ce qui va faciliter cette insertion car l'implant sera tout le long de cette composante curviligne de la trajectoire accouplé et tenu par ce porte-implant.

Selon une caractéristique, la partie arrière présente deux faces latérales opposées, respectivement une face supérieure et une face inférieure, et les moyens de pivotement sont prévus sur lesdites faces latérales opposées sous la forme d'évidements ou de protubérances conformés au moins partiellement en arc de cercle centré sur l'axe transversal.

Ainsi, les moyens de pivotement fonctionnent par coopération de forme, les moyens de pivotement complémentaires prévus sur le porte-implant étant de formes complémentaires pour un accouplement par coopération de forme qui autorise un pivotement relatif entre l'implant et le porte-implant.

Dans un mode de réalisation particulier, la partie avant comprend des facettes reliées directement entre elles par des arêtes vives convergeant vers l'extrémité avant fuselée en pointe.

L'avantage procuré par les arêtes vives est de pouvoir perforer et trancher le ligament interépineux aisément et frayer un chemin à l'implant.

Selon une variante, la pointe et les facettes de la partie avant définissent un volume de forme tronconique, pyramidale ou trapézoïdale. Un avantage procuré par une telle forme est de faciliter la perforation des zones ligamentaires tout en restant compact. Selon une variante de l'invention, les arêtes vives peuvent être obtenues par un profilé selon un simple usinage.

Selon une variante de l'invention, au moins une des arêtes vives et préférentiellement toutes les arêtes vives sont rectilignes, ce qui permet la perforation nette des zones ligamentaires.

Dans une réalisation avantageuse, la partie arrière comprend, à une interface avec la partie centrale, au moins une ailette de blocage faisant saillie sur un côté inférieur de la partie arrière ou sur un côté supérieur de la partie arrière, opposé au côté inférieur. Aussi, la ou chaque ailette de blocage s'étend à l'avant de la partie arrière, et donc à l'arrière de la partie centrale, entre la partie arrière et la partie centrale, et elle forme une ailette propre à venir en butée contre une apophyse épineuse, de sorte à stopper l'implant dans sa trajectoire d'insertion et assurer un positionnement précis entre les deux apophyses épineuses. Ainsi, lors de la pose de l'implant, la ou chaque ailette de blocage interdit le déplacement de l'implant hors de l'espace interépineux en se plaquant chacune contre une apophyse épineuse adjacente, afin d'éviter le retrait involontaire de l'implant.

Avantageusement, la partie arrière comprend, à l'interface avec la partie centrale, une ailette de blocage faisant saillie sur le côté inférieur de la partie arrière et une autre ailette de blocage faisant saillie sur le côté supérieur de la partie arrière.

Selon une variante, les deux ailettes de blocage sont coplanaires.

Selon une autre variante, les deux ailettes de blocage sont identiques et symétriques par rapport à un plan médian contenant l'axe longitudinal.

Selon une possibilité, l'au moins une ailette de blocage s'étend depuis le côté concerné de la partie arrière selon un plan orthogonal à l'axe longitudinal ou incliné par rapport à l'axe longitudinal d'un angle compris entre 60 et 120 degrés.

Ainsi, lorsque la ou chaque ailette de blocage s'étend depuis la partie arrière selon un plan sensiblement perpendiculaire à l'axe longitudinal du corps d'implant, elle s'ancre naturellement sur l'apophyse épineuse correspondante.

Selon une autre possibilité, l'au moins une ailette de blocage est fixe ou articulée sur la partie arrière.

L'avantage d'une ailette de blocage fixe est d'avoir un corps d'implant de conception aisée et peu coûteuse.

Dans le cas où l'ailette de blocage est articulée, celle-ci est alors mobile entre au moins une position déployée dans laquelle elle s'étend selon un axe sensiblement perpendiculaire à l'axe longitudinal du corps d'implant et une position repliée dans laquelle dans laquelle elle s'étend, préférentiellement contre le côté concerné de la partie arrière, selon un axe sensiblement parallèle, à l'axe longitudinal du corps d'implant.

Selon une autre possibilité, l'au moins une ailette de blocage présente une face avant tournée en direction de l'extrémité avant et sur laquelle est prévu au moins un relief d'ancrage configuré pour permettre un ancrage sur une apophyse épineuse, comme par exemple un relief d'ancrage en forme de picot.

Cette face avant est destinée à être en regard de l'un des apophyses épineuses, et le ou les reliefs d'ancrage vont apporter un ancrage stable dans cette apophyse épineuse dans la position finale de l'implant.

Selon une variante, l'au moins une ailette de blocage présente plusieurs reliefs d'ancrage.

Selon une autre variante, le au moins un picot s'étend selon un axe sensiblement parallèle à l'axe longitudinal de l'implant.

Selon une autre variante, la face avant de l'au moins une ailette de blocage est revêtue d'un matériau d'ostéo-intégration, favorisant l'adhésion entre l'os et le corps d'implant, comme par exemple un matériau poreux.

Dans un mode de réalisation particulier, la partie centrale présente deux faces latérales opposées, respectivement une face supérieure et une face inférieure, qui sont concaves pour définir deux faces latérales en creux propres à s'intercaler entre les deux apophyses épineuses.

Il est à noter que la face supérieure de la partie centrale est prévue pour être en appui sur l'apophyse de la vertèbre sus-jacente, tandis que la face inférieure de la partie centrale est prévue pour être en appui sur l'apophyse de la vertèbre sous-jacente. Ainsi, cette forme en creux de ces deux faces permet d'épouser l'embase des deux apophyses épineuses et favorise également une légère déformation élastique propre à assurer une distraction souple entre les apophyses épineuses adjacentes, l'une par rapport à l'autre et ainsi de réduire la lordose à ce niveau.

Selon une variante, le corps d'implant est réalisé dans au moins un matériau stérilisable biocompatible. A titre d'exemples non limitatifs, le corps d'implant peut être réalisé dans un matériau sélectionné parmi le titane, un alliage à base de titane, un acier, un matériau plastique biocompatible tel que le PolyEtherEtherKethone (PEEK), le polyméthacrylate de méthyle (PMMA), etc.

L'invention se rapporte également à un ancillaire de pose, prévu pour un guidage et un positionnement d'un implant vertébral interépineux selon l'invention entre deux apophyses épineuses de deux vertèbres adjacentes, cet ancillaire de pose comprenant :
- un porte-implant présentant une partie distale et une partie proximale opposées, ladite partie distale étant munie de moyens de support conformés pour supporter ledit implant vertébral interépineux ;
- une broche d'implantation présentant une portion de terminaison distale qui s'étend jusqu'à une extrémité distale libre et en forme de pointe, ladite portion de terminaison distale présentant une forme incurvée et sur laquelle est prévu un rail de guidage incurvé et de forme complémentaire à la rainure antérieure de l'implant vertébral interépineux pour permettre un guidage en coulissement dudit implant vertébral interépineux le long d'une face postérieure de ladite portion de terminaison distale par engagement du rail de guidage à l'intérieur de la rainure antérieure, ledit rail de guidage s'étendant jusqu'à l'extrémité distale libre.

Ainsi, l'implant vertébral interépineux va pouvoir être guidé par le rail de guidage de cette broche d'implantation incurvée, selon une trajectoire au moins partiellement curviligne qui permet une insertion intuitive et progressive entre les deux apophyses épineuses des vertèbres. En d'autres termes, l'implant, avec sa rainure antérieure, va pouvoir se positionner et coulisser sur la broche d'implantation incurvée pour autoriser une intervention selon une voie d'abord majoritairement dorsale, selon une trajectoire curviligne (permise grâce à forme de la broche d'implantation incurvée) et en faisant appel à une ouverture dorsale, légèrement excentrée de l'axe dorsal, favorisant ainsi une implantation directe dans la zone d'implantation déterminée.

Autrement dit, grâce à un tel ancillaire de pose selon l'invention, la broche d'implantation est orientée de manière incurvée sur sa portion de terminaison distale, ce qui autorise une intervention chirurgicale selon une voie d'abord majoritairement dorsale. En outre, du fait de sa position antérieure par rapport à l'implant, la broche d'implantation vient protéger les éléments nerveux présents dans le canal vertébral.

Il est à noter que, au sens de l'invention, la broche d'implantation forme un corps longiligne, en particulier de forme courbe, se terminant par une extrémité distale libre et en forme de pointe, de manière à pouvoir pénétré, en se frayant un chemin à travers les tissus, dans le corps du patient jusqu'à une position adéquate en regard de l'espace interépineux, entre les deux apophyses épineuses, ce qui relève d'une fonction classique d'une broche dans le domaine chirurgical. Par ailleurs, cette broche d'implantation présente la particularité précédemment décrite, à savoir la présence d'un rail de guidage sur lequel l'implant vertébral interépineux est guidé en coulissement ; ce coulissement s'opérant nécessairement sur la broche d'implantation (autrement dit l'implant est à l'extérieur de la broche d'implantation), et non pas à l'intérieur de la broche d'implantation, car dans le domaine chirurgical, il est bien entendu qu'une broche ne forme pas une goulotte ou un tube à l'intérieur de laquelle pourrait glisser un implant.

Il est également à noter que le rail de guidage prévu sur la broche d'implantation a la fonction de guider l'implant vertébral interépineux selon une trajectoire courbe d'implantation, mais il a aussi nécessairement la fonction d'empêcher ou limiter un débattement latéral de l'implant pour éviter qu'il ne sorte du rail de guidage.

Avantageusement, le positionnement de la portion de terminaison distale incurvée de la broche d'implantation se fera le plus antérieurement possible au niveau de l'espace interépineux car la situation optimale de l'implant en fin d'intervention correspond à la partie antérieure de l'espace inter-épineux, zone où l'os est le plus large et le plus solide. La direction curviligne de la rainure de guidage est ainsi choisie de sorte que la broche d'implantation présente un angle d'attaque satisfaisant pour accéder à l'espace interépineux.

Dans la présente invention, la notion de « proximale » se réfère à une partie ou à une extrémité la plus proche de la main du chirurgien lors de la pose de l'implant, et la notion de « distale » se réfère à une partie ou à une extrémité la plus éloignée de la main du chirurgien lors de la pose, ou autrement dit la plus proche du patient au moment de l'intervention.

Selon une caractéristique, la broche d'implantation est pleine, et elle ne forme pas un tube ou une goulotte.

Selon une possibilité, la portion de terminaison distale de la broche d'implantation est incurvée en arc de cercle sur un secteur angulaire compris entre 60 et 120 degrés.

Dans une réalisation particulière, la portion de terminaison distale de la broche d'implantation est incurvée en arc de cercle selon un rayon de courbure compris entre 20 et 300 millimètres.

Un avantage procuré par de tels rayons de courbure est de faciliter l'intervention chirurgicale selon une voie d'abord majoritairement dorsale, et de limiter la taille de l'incision.

Selon une autre possibilité, la broche d'implantation comprend une portion de terminaison proximale qui prolonge la portion de terminaison distale jusqu'à une extrémité proximale libre, et le rail de guidage est prolongé, à partir de la portion de terminaison distale, par un rail longitudinal ménagé sur la portion de terminaison proximale jusqu'à l'extrémité proximale.

Ainsi, l'implant vertébral interépineux peut être glissé sur le rail longitudinal au niveau de l'extrémité proximale, pour coulisser en direction de la portion de terminaison distale incurvée afin de passer dans le rail de guidage incurvé pour coulisser et suivre une trajectoire curviligne.

Il est clair que, au sens de l'invention, le rail de guidage et le rail longitudinal peuvent former qu'un seul et même rail continu sur lequel peut s'engager l'implant vertébral interépineux depuis l'extrémité proximale jusqu'à l'extrémité distale.

Autrement dit, le rail de guidage et le rail longitudinal sont prévus ensemble sur la broche d'implantation avec la fonction de guider l'implant vertébral interépineux selon une trajectoire complète d'implantation rectiligne puis incurvée, mais ce rail de guidage et ce rail longitudinal ont aussi nécessairement la fonction d'empêcher ou limiter un débattement latéral de l'implant pour éviter qu'il ne sorte du rail de guidage et du rail longitudinal.

Selon une possibilité, l'un au moins de la portion de terminaison proximale et du rail longitudinal est rectiligne, autrement dit cette portion de terminaison proximale est rectiligne et/ou ce rail longitudinal est rectiligne.

Ainsi, l'implant vertébral interépineux suivra une trajectoire d'insertion ayant d'abord une composante linéaire (glissement sur le rail longitudinal) puis enfin une composante curviligne (glissement sur le rail de guidage incurvé), ce qui permet à l'implant de passer d'une orientation verticale (à l'extérieur du patient) à une orientation horizontale (à l'intérieur du patient dans l'espace interépineux).

Selon une caractéristique, le rail de guidage est ménagé sur au moins une face externe de la portion de terminaison distale de la broche d'implantation.

Autrement dit, le rail de guidage est ménagé sur l'extérieur de la broche d'implantation, pour un coulissement de l'implant sur la broche d'implantation (et non pas à l'intérieur d'un tube ou d'une goulotte).

Selon une autre caractéristique, l'extrémité distale libre et en forme de pointe de la broche d'implantation présente un angle de pointe inférieure ou égale à 90 degrés, et de préférence inférieure ou égale à 60 degrés, voire inférieure ou égale à 45 degrés. En effet, un angle de pointe faible favorise la pénétration de la broche d'implantation à travers les tissus.

Dans une réalisation particulière, la portion de terminaison distale de la broche d'implantation présente deux faces latérales opposées, respectivement une face supérieure et une face inférieure, et le rail de guidage est ménagé sous la forme de deux encoches ménagées dans lesdites faces latérales respectives.

De telles encoches sont adaptées pour recevoir respectivement les lèvres de retenue décrites précédemment et ainsi procurer un guidage stable et fiable sur toute la distance.

Selon une caractéristique, la portion de terminaison distale de la broche d'implantation présente une face antérieure, opposée à la face postérieure le long de laquelle coulisse l'implant vertébral interépineux, et sur laquelle est prévu un premier moyen de guidage en pivotement,
et l'ancillaire de pose comprend un support primaire présentant une partie distale sur laquelle sont prévus au moins un relief d'ancrage pour un ancrage dans une vertèbre, et un second moyen de guidage en pivotement propre à coopérer avec le premier moyen de guidage en pivotement pour guider ladite broche d'implantation en pivotement lors d'une insertion de sa portion de terminaison distale entre les deux apophyses épineuses.

Ainsi, ce support primaire va être ancré dans une vertèbre, dans une zone se situant dans un plan médian passant entre les deux apophyses épineuses, et ensuite ce support primaire va servir à diriger la broche d'implantation pour amener sa portion de terminaison distale entre les deux apophyses épineuses, selon un mouvement de pivotement ou de basculement de la broche d'implantation.

Dans un mode de réalisation particulier, le premier moyen de guidage en pivotement et le second moyen de guidage en pivotement comprennent respectivement un rail arqué et une glissière arquée de formes complémentaires.

Selon une variante, le premier moyen de guidage en pivotement et le second moyen de guidage en pivotement sont conformés pour guider la broche d'implantation en pivotement selon un angle de pivotement compris entre 60 et 120 degrés, et notamment de l'ordre de 90 degrés.

Selon une autre caractéristique, le support primaire présente une partie proximale accouplé à une poignée de maintien muni d'une tête de préhension.

Une telle poignée de maintien a pour fonction principale de permettre une préhension par le chirurgien et donc pour permettre au chirurgien une mise en place et un maintien du support primaire afin de l'ancrer dans la vertèbre, notamment en exerçant un effort de poussée, et si besoin un effort d'impaction sur la tête de préhension, de préférence répétée, pour enfoncer le support primaire.

Selon une variante, la poignée de maintien est distincte du support primaire et est solidarisée au support primaire ou la poignée de maintien et le support primaire sont réalisés d'un seul tenant.

Selon autre caractéristique, le support primaire présente un trou distal débouchant dans une face distale de la partie distale du support primaire, et l'ancillaire de pose comprend un pointeau de positionnement muni d'une pointe prévue pour un ancrage dans une vertèbre, ledit pointeau de positionnement étant conformé pour guider longitudinalement en coulissement le support primaire par insertion du pointeau de positionnement à l'intérieur du trou distal.

Ainsi, ce pointeau de positionnement va être ancré dans une vertèbre, à un point précis déterminé par le chirurgien et se situant sensiblement dans le plan médian passant entre les deux apophyses épineuses, et il servira à guider le support primaire pour le positionner dans la zone souhaitée.

Dans un mode de réalisation particulier, les moyens de support prévus sur la partie distale du porte-implant comprennent des moyens de pivotement complémentaires conformés pour coopérer avec les moyens de pivotement de la partie arrière de l'implant vertébral interépineux afin d'autoriser un pivotement de cet implant vertébral interépineux selon un axe transversal.

Selon une possibilité, les moyens de support comprennent deux éléments de support présentant des faces internes en vis-à-vis munies respectivement d'évidements ou de protubérances conformés au moins partiellement en arc de cercle centré sur l'axe transversal et formant les moyens de pivotement complémentaires.

Ainsi, l'implant vertébral interépineux est coincé entre les faces internes des deux éléments de support respectifs, tout en étant mobile en pivotement entre ces deux faces internes.

En outre, les moyens de pivotement fonctionnent par coopération de forme, les moyens de pivotement complémentaires prévus sur le porte-implant étant de formes complémentaires pour un accouplement par coopération de forme qui autorise un pivotement relatif entre l'implant et le porte-implant.

Selon une autre possibilité, les deux éléments de support sont attachés l'un à l'autre de manière détachable et, à cet effet, sont munis de moyens d'attachement amovible afin que les deux éléments de support soient configurables entre une configuration attachée pour pouvoir supporter l'implant vertébral interépineux, et une configuration détachée pour pouvoir libérer l'implant vertébral interépineux du porte-implant.

De cette manière, une fois que l'implant vertébral interépineux est en place entre les deux apophyses épineuses, il suffit de détacher les deux éléments de support entre eux pour pouvoir libérer l'implant vertébral interépineux et ainsi retirer le porte-implant tout en laissant l'implant vertébral interépineux en place.

Selon une possibilité, les moyens d'attachement amovible sont formés d'une glissière supérieure et d'un rail supérieur qui sont prévus sur les éléments de support respectifs.

Selon une autre possibilité, la glissière supérieure et le rail supérieur sont tous deux arqués et de formes complémentaires et s'étendent entre une face antérieure et une face postérieure de la partie distale du porte-implant, pour former des moyens de guidage en pivotement propres à coopérer ensemble pour guider en pivotement un élément de support relativement à l'autre élément de support selon l'axe transversal. Ainsi, le détachement entre les deux éléments de support se fait par pivotement, sans gêner l'implant.

Selon une autre possibilité, la partie distale et la partie proximale du porte-implant sont reliées par deux tiges, ces tiges présentant des extrémités distales fixées sur les éléments de support respectifs et des extrémités proximales, opposées à leurs extrémités distales, qui sont fixées de manière amovible sur la partie proximale.

Avantageusement, lequel les extrémités distales des tiges présentent des terminaisons respectives qui dépassent des éléments de support respectifs, afin de pouvoir venir en appui contre des ailettes de blocage respectives de l'implant.

Dans un mode de réalisation particulier, l'ancillaire de pose comprend un impacteur monté de manière amovible sur la partie proximale du porte-implant.

Selon une caractéristique, l'impacteur comprend une tige d'impaction munie d'une extrémité distale fixée sur la partie proximale du porte-implant, et d'une extrémité distale, opposée à l'extrémité distale, et sur laquelle est fixée une poignée.

Selon une autre caractéristique, l'impacteur comprend en outre un manchon d'impaction monté coulissant autour de la tige d'impaction, entre la poignée et la partie proximale du porte-implant.

D'autres caractéristiques et avantages de la présente invention apparaîtront à la lecture de la description détaillée ci-après, d'un exemple de mise en œuvre non limitatif, faite en référence aux figures schématiques annexées dans lesquelles :
[Fig. 1] est une illustration en perspective sur le côté postérieur d'un exemple d'implant vertébral interépineux selon l'invention ;
[Fig. 2] est une illustration de face (côté avant) de l'implant de la Figure 1 ;
[Fig. 3] est une illustration de dessous (côté face intérieure) de l'implant de la Figure 1 ;
[Fig. 4] est une illustration en perspective sur le côté antérieur de l'implant de la Figure 1 ;
[Fig. 5] est une illustration de dos (côté arrière) de l'implant de la Figure 1 ;
[Fig. 6] est une illustration du côté postérieur de l'implant de la Figure 1 ;
[Fig. 7] est une illustration du côté antérieur de l'implant de la Figure 1 ;
[Fig. 8] est une illustration en perspective (vue de dos et du côté postérieur) de l'implant de la Figure 1 en situation entre deux apophyses épineuses ;
[Fig. 9] est une vue est une illustration en perspective (vue de face et du côté postérieur) de l'implant de la Figure 1 en situation entre deux apophyses épineuses ;
[Fig. 10] est une illustration du côté postérieur de l'implant de la Figure 1 en situation entre deux apophyses épineuses ; et
[Fig. 11] est une illustration d'une première étape d'insertion d'un pointeau de positionnement de l'ancillaire de pose selon l'invention ;
[Fig. 12] est une illustration d'une deuxième étape d'insertion d'un support primaire de l'ancillaire de pose selon l'invention ;
[Fig. 13] est une illustration en perspective de la partie distale du support primaire de la Figure 12, selon deux angles de vue distincts ;
[Fig. 14] est une illustration d'une troisième étape d'accouplement d'une poignée de maintien sur le support primaire, et d'une quatrième étape d'insertion d'une broche d'implantation de l'ancillaire de pose selon l'invention ;
[Fig. 15] est une illustration en perspective de la poignée de maintien seule de la Figure 14 ;
[Fig. 16] est une illustration zoomée et en perspective de la partie distale du support primaire sur laquelle est guidée la portion de terminaison distale de la broche d'implantation lors de la quatrième étape de la Figure 14 ;
[Fig. 17] est une illustration en perspective de la broche d'implantation seule de la Figure 14, selon plusieurs angles de vue et en entier (la vue de gauche) ou partielle (les trois vues de droite) ;
[Fig. 18] est une illustration d'une cinquième étape de pivotement (ou basculement) de la broche d'implantation relativement au support primaire statique ;
[Fig. 19] est une illustration en perspective d'un ensemble de l'ancillaire de pose selon l'invention, comprenant un porte-ancillaire supportant l'implant de la Figure 1, et comprenant en outre un impacteur monté sur le porte-implant ;
[Fig. 20] est une illustration en perspective du porte-implant seul de la Figure 19 ;
[Fig. 21] est une illustration en perspective de la partie distale du porte-implant de la Figure 19, avec ses deux éléments de support en configuration attachée (à gauche) et en configuration détachée (à droite) ;
[Fig. 22] est une illustration d'une sixième étape d'insertion de l'ensemble porte-ancillaire et impacteur de la Figure 19, le porte-ancillaire supportant l'implant de la Figure 1 ;
[Fig. 23] est une illustration zoomée et en perspective de la partie distale du support primaire et de la portion de terminaison distale de la broche d'implantation sur laquelle coulisse l'implant porté par le porte-implant de la Figure 19 ;
[Fig. 24] est une illustration zoomée et en perspective de la partie distale du support primaire et de la portion de terminaison distale de la broche d'implantation sur laquelle coulisse l'implant porté par le porte-implant de la Figure 19 ;
[Fig. 25] est une illustration du début d'une septième étape de poussée de l'implant de la Figure 1 qui coulisse sur la broche d'implantation en direction de l'espace interépineux entre les deux apophyses épineuses ;
[Fig. 26] est une illustration zoomée et en perspective de l'implant qui coulisse sur la broche d'implantation au début de la septième étape de la Figure 25 ;
[Fig. 27] est une illustration de la fin de la septième étape de poussée de l'implant de la Figure 1 qui a coulissé sur la broche d'implantation et qui est désormais positionné entre les deux apophyses épineuses ;
[Fig. 28] est une illustration zoomée et en perspective de l'implant sur la broche d'implantation à la fin de la septième étape de la Figure 27 ;
[Fig. 29] est une illustration d'une huitième étape de retrait de l'impacteur vis-à-vis du porte-implant ;
[Fig. 30] est une illustration d'une neuvième étape de déverrouillage du porte-implant en faisant passer ses deux éléments de support en configuration détachée ;
[Fig. 31] est une illustration zoomée et en perspective de l'implant et du porte-implant lors de la neuvième étape de la Figure 30 ; et
[Fig. 32] est une illustration d'une dixième étape de retrait de la broche d'implantation, après avoir retiré le porte-implant suite à la neuvième étape de la Figure 30.

Les éléments identiques ou assurant la même fonction porteront les mêmes références pour les différents modes de réalisation, par souci de simplification.

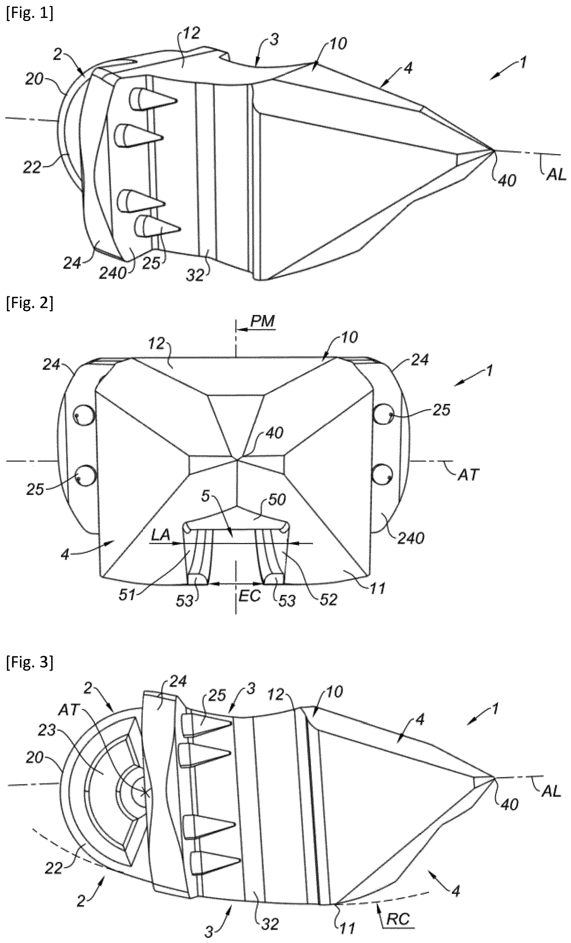

L'implant 1 selon l'invention va maintenant être décrit en référence aux figures 1 à 7. Cet implant 1 selon l'invention est un implant vertébral interépineux comportant un corps d'implant 10 réalisé dans au moins un matériau biocompatible. Plus précisément, cet implant 1 est formé intégralement et uniquement de ce corps d'implant 10.

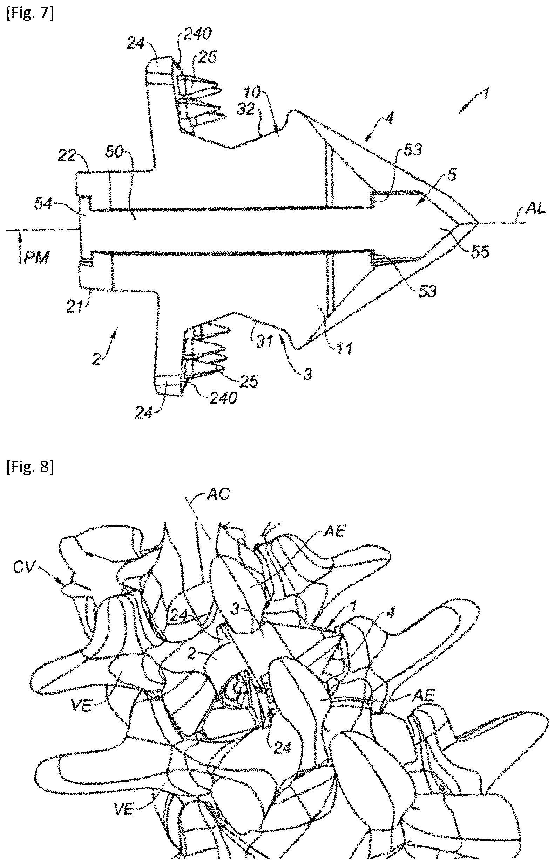

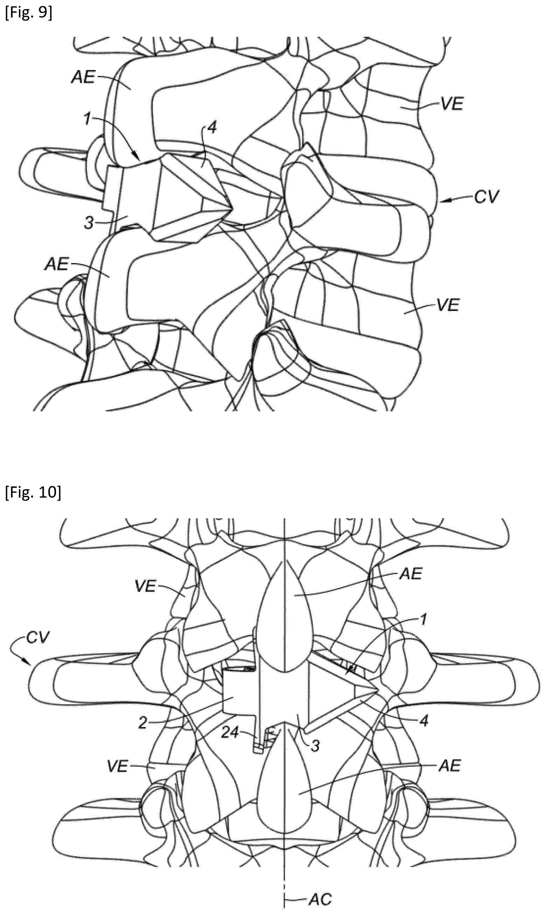

Ce corps d'implant 10 s'étendant selon un axe longitudinal AL et il comprend successivement le long de cet axe longitudinal AL :
- une partie arrière 2 présentant une extrémité arrière 20,
- une partie centrale 3 conformée pour s'étendre entre deux apophyses épineuses AE (comme visible sur les Figures 8 à 10) de deux vertèbres VE adjacentes, cette partie centrale 3 prolongeant la partie arrière 2, et
- une partie avant 4 prolongeant la partie centrale 3, de manière opposée à la partie arrière 3, en s'effilant jusqu'à une extrémité avant 40 de forme fuselée.

Ainsi, la partie centrale 3 relie la partie arrière 2 à la partie avant 4, et cette partie centrale 3 s'étend entre la partie arrière 2 à la partie avant 4.

Il est à noter que la partie centrale 3 peut présenter une certaine élasticité, comparativement à la partie avant 2, autorisant une flexion de la partie centrale 3 lors de sa trajectoire d'insertion, en particulier durant la composante curviligne de cette trajectoire d'insertion. Une fois insérée, cette flexibilité du corps d'implant 1 permet de préserver la mobilité entre les deux vertèbres adjacentes dans une plage donnée. Cette élasticité de la partie centrale 3, qui contribue à avoir un corps d'implant 10 avec deux "zones" de rigidité/flexibilité différentes, peut par exemple être obtenue par des modifications géométriques (eg. structure type treillis) et/ou par l'emploi de matériaux différents.

La partie arrière 2 présente une forme générale de demi-disque avec son extrémité arrière 20 qui est arrondie ou plutôt de forme semi-cylindrique centré sur un axe transversal AT qui est perpendiculaire à l'axe longitudinal AL.

Cette partie arrière 2 présente deux faces latérales 21, 22 opposées, respectivement une face supérieure 21 et une face inférieure 22, en forme générale de demi-disque, et des moyens de pivotement selon l'axe transversal AT sont prévus sur ces deux faces latérales 21, 22. Pour ce faire, les moyens de pivotement sont prévus sous la forme d'évidements 23 formés dans les faces latérales 21, 22 respectives et conformés au moins partiellement en arc de cercle centré sur l'axe transversal AT. Comme visible en Figure 3, un évidement 23 se présente sous la forme d'un évidement en arc de cercle selon un secteur angulaire inférieur à 180 degrés, comme par exemple et à titre non limitatif un secteur angulaire compris entre 20 et 175 degrés.

Comme expliqué ultérieurement, ces évidements 23 sont prévus pour coopérer avec des protubérances 941, 951 complémentaires prévus sur un porte-implant 9 pour autoriser un pivotement relatif du corps d'implant 10 sur ce porte-implant 9 selon l'axe transversal AT.

A la jonction entre la partie arrière 2 et la partie centrale 3, la partie arrière 2 comprend une ailette de blocage 24 faisant saillie sur le côté inférieur de la partie arrière 2 (du même côté que la face inférieure 22) et une autre ailette de blocage 24 faisant saillie sur le côté supérieur de la partie arrière 2 (du même côté que la face supérieure 21).

Les deux ailettes de blocage 24 sont symétriques par rapport à un plan médian PM incluant l'axe longitudinal AL et orthogonal à l'axe transversal AT, et l'ailette de blocage 24 s'étend perpendiculairement à la face inférieure 22 tandis que l'autre ailette de blocage 24 s'étend sensiblement perpendiculairement à la face supérieure 21.

Ces ailettes de blocage 24 sont coplanaires en s'étendant dans un plan sensiblement orthogonal à l'axe longitudinal AL, ou plus généralement dans un plan incliné par rapport à l'axe longitudinal AL d'un angle compris entre 60 et 120 degrés. De manière générale, le corps d'implant 10 est symétrique par rapport à ce plan médian PM.

Les deux ailettes de blocage 24 saillent extérieurement par rapport aux faces latérales 21, 22 respectives, et aussi par rapport à la partie centrale 3. Les deux ailettes de blocage 24 sont fixes et viennent de matière avec le corps d'implant 10. Dans une variante non illustrée, les ailettes de blocage 24 sont articulées sur les faces latérales 21, 22 respectives ou les ailettes de blocage 24 sont montées de manière amovible sur les faces latérales 21, 22.

Les deux ailettes de blocage 24 présentent chacune une face avant 240 tournée vers l'extrémité avant 40, et une face arrière 241 opposée à la face avant 240 et tournée vers l'extrémité arrière 20.

Sur chacune des faces avant 240 des deux ailettes de blocage 24 sont prévus plusieurs picots 25 formant des reliefs d'ancrage configurés pour permettre un ancrage sur une apophyse épineuse AE. Ces picots 25 s'étendent selon des directions parallèles à l'axe longitudinal AL. Les faces arrières 241 des deux ailettes de blocage 24 sont quant à elles lisses.

La partie centrale 3 présente deux faces latérales 31, 32 opposées, respectivement une face supérieure 31 et une face inférieure 32, qui sont concaves pour définir deux faces latérales 31, 32 en creux propres à s'intercaler entre les deux apophyses épineuses AE. Autrement dit, la partie centrale 3 est amincie en son centre pour épouser la forme des apophyses épineuses AE. Les deux ailettes de blocage 24 saillent extérieurement par rapport aux faces latérales 31, 32 respectives.

La partie avant 4 présente une forme fuselée adaptée pour perforer des zones ligamentaires, et pour cela elle comprend des facettes planes reliées directement entre elles par des arêtes vives et rectilignes convergeant vers l'extrémité avant 40 fuselée en pointe.

Le corps d'implant 10 présente aussi une face antérieure 11 destinée à être orientée vers les vertèbres VE, et une face postérieure 12, opposée à la face antérieure 11.

La face antérieure 11 présente est de forme convexe selon un rayon de courbure RC compris entre 20 et 300 millimètres, avec un centre de courbure situé en regard de la face postérieure 12 du corps d'implant 10. La face postérieure 12 présente quant à elle une forme convexe au niveau de la partie centrale 3 et une forme de facette plane au niveau de la partie avant 4.

Le corps d'implant 10 présente également une rainure antérieure 5 ménagée dans la face antérieure 11 et traversant en totalité le corps d'implant 10 de son extrémité arrière 20 jusqu'à son extrémité avant 40 pour former, comme décrit ultérieurement, un canal de guidage conformé pour recevoir au moins une portion d'une broche d'implantation 8 d'un ancillaire de pose décrit ci-après.

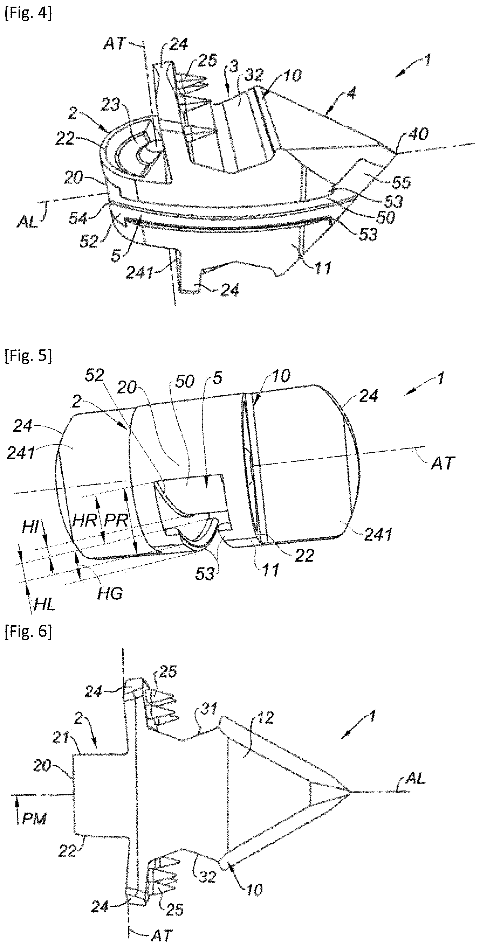

Comme clairement visible sur les Figures 4 et 7, cette rainure antérieure 5 est ménagée de manière continue dans la face antérieure 11 du corps d'implant 10 depuis l'extrémité arrière 20 jusqu'à l'extrémité avant 40, de manière à déboucher continuellement dans la face antérieure 11.

Cette rainure antérieure 5 est de forme convexe selon le rayon de courbure RC, et s'étend donc depuis l'extrémité arrière 20 jusqu'à l'extrémité avant 40 suivant une direction curviligne en arc de cercle. Cette rainure antérieure 5 s'étend dans le plan médian PM et elle est symétrique par rapport au plan médian PM.

Bien qu'une certaine flexibilité soit envisageable pour le corps d'implant 10, ce corps d'implant 10 ne forme en aucun cas un corps articulé, autrement dit ce corps d'implant 10 est non articulé, dans le sens où dans une position de repos (position visible sur les Figures 1 à 7) comme dans une position implantée (position visible sur les Figures 8 à 10 et 27 à 32), la rainure antérieure 5 est de forme curviligne selon une même courbure.

La rainure antérieure 5 est délimitée intérieurement par une paroi de fond 50 décalée en profondeur par rapport à la face antérieure d'une profondeur PR donnée constante depuis l'extrémité arrière 20 jusqu'à l'extrémité avant 40, comme visible en Figure 5.

Cette paroi de fond 50 est de forme convexe avec un centre de courbure confondu avec le centre de courbure de la face antérieure 11 et ayant un rayon de courbure RP (non illustré) inférieur au rayon de courbure RC de cette face antérieure 11. La profondeur PR est mesurée selon une direction radiale passant par le centre de courbure, de sorte que RC = RP + PR.

Cette rainure antérieure 5 est aussi délimitée intérieurement par deux parois latérales 51, 52 parallèles au plan médian PM et en forme d'arc de cercle. L'écartement entre les deux parois latérales 51, 52 définit la largeur LA de la rainure antérieure 5.

La rainure antérieure 5 est délimitée extérieurement par des lèvres de retenue 53 de forme convexe selon le rayon de courbure RC au niveau de la face antérieure 11. Ces lèvres de retenue 53 saillent des parois latérales 51, 52 respectives, par ailleurs elles sont disposées en regard de la paroi de fond 50 et elles s'étendent l'une en face de l'autre pour délimiter un espace rétréci comparativement à l'intérieur de la rainure antérieure 5. Autrement dit, l'écartement EC entre les deux lèvres de retenue 53 est inférieur à la largeur LA de la rainure antérieure 5. Ces lèvres de retenue 53 font partie intégrante de la face antérieure 11 dans laquelle débouche la rainure antérieure 5. Les lèvres de retenue 53 présentent chacune une épaisseur ou hauteur de lèvre HL, comme visible en Figure 5, mesurée selon une direction radiale passant par le centre de courbure. Ainsi, la rainure antérieure 5 présente une hauteur de rainure HR, également mesurée selon une direction radiale passant par le centre de courbure, entre les lèvres de retenue 53 et la paroi de fond 50, qui est telle que : PR = HR + HL.

Par ailleurs, la rainure antérieure 5 est conformée de sorte que :
- en considérant une première directrice et une deuxième directrice parallèles à l'axe longitudinal AL, où la première directrice passe par le point le plus bas de la paroi de fond 50 (sensiblement à mi-chemin entre l'extrémité arrière 20 et l'extrémité avant 40) et la deuxième directrice passe par le point les plus hauts des lèvres de retenue 53 (au niveau de l'extrémité arrière 20 ou de l'extrémité avant 40), alors la rainure antérieure 5 présente une hauteur intermédiaire HI non nulle, qui correspond à une distance mesurée entre la première directrice et la deuxième directrice selon une direction orthogonale à l'axe longitudinal AL et à l'axe transversal AT, comme visible sur la Figure 5 ; et
- en considérant une troisième directrice parallèle à l'axe longitudinal AL, où la troisième directrice passe par le point le plus bas des lèvres de retenue (sensiblement à mi-chemin entre l'extrémité arrière 20 et l'extrémité avant 40), alors les lèvres de retenue 53 présentent une hauteur globale HG, qui correspond à une distance mesurée entre la deuxième directrice et la troisième directrice selon une direction orthogonale à l'axe longitudinal AL et à l'axe transversal AT, comme visible sur la Figure 5

Une telle conformation est avantageuse pour permettre de faire coulisser l'implant 1 sur un rail longitudinal 89 (décrit ultérieurement) selon une trajectoire longitudinale (autrement dit rectiligne) puis sur un rail de guidage 83 incurvé (décrit ultérieurement) selon une trajectoire courbée ou arquée.

La rainure antérieure 5 présente à ses deux extrémités respectives une bouche arrière 54 ouverte sur l'extrémité arrière 20, et une bouche avant 55 ouverte sur l'extrémité avant 40.

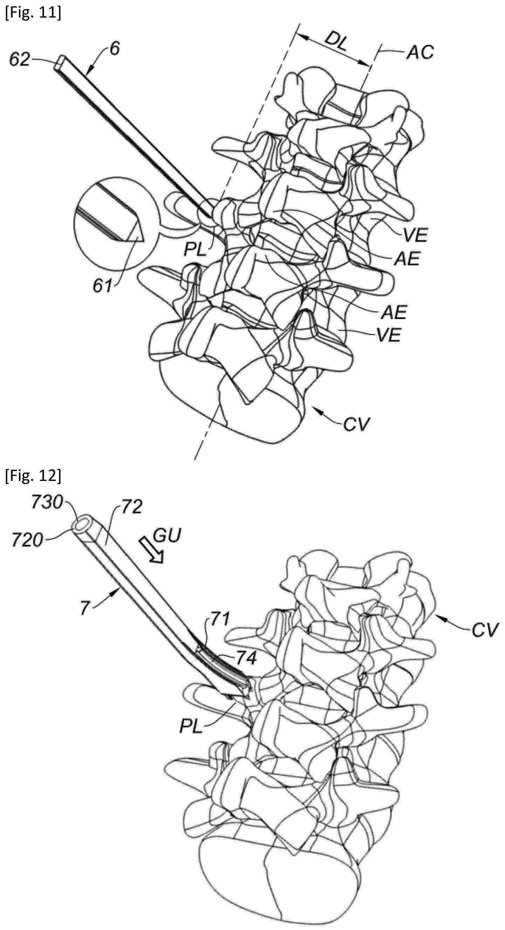

La suite de la description, en référence aux Figures 11 à 32, porte sur l'ancillaire de pose prévu pour un guidage et un positionnement de l'implant 1 entre deux apophyses épineuses AE de vertèbres VE adjacentes, ainsi que sur la méthode de pose de l'implant 1 au moyen d'un tel ancillaire de pose.

Cet ancillaire de pose se présente sous la forme d'un kit comprenant plusieurs composants fonctionnels distincts et coopérant ensemble pour permettre une implantation selon une voie d'abord majoritairement dorsale de l'implant 1. Ces différents composants de l'ancillaire de pose seront décrits au fur et à mesure de leur utilisation dans la mise en œuvre de la méthode de pose.

En préalable et en référence à la Figure 11, le chirurgien détermine un point de référence localisé dans une partie latérale PL d'une vertèbre VE, à une distance latérale DL par rapport à l'axe AC de la colonne vertébrale CV (aussi appelé axe dorsal), une telle distance latérale DL étant déterminée préalablement avant l'opération - par exemple entre 5 et 70 millimètres. Ce point de référence se situe substantiellement dans un plan médian passant entre les deux apophyses épineuses AE. Ainsi, la méthode d'implantation est dite d'abord majoritairement dorsal, dans le sens où elle fait appel à une approche dorsale mais excentrée légèrement de l'axe dorsal de la distance latérale DL.

Cette étape préalable de détermination du point de référence peut être réalisée à l'avance par exemple par l'usage d'un scanner préopératoire, le scanner préopératoire permettant également de mesurer la largeur de l'espace interépineux ciblé et de choisir une taille de l'implant 1 en fonction de cette largeur.

Ensuite, le chirurgien effectue une incision à la distance latérale DL par rapport à l'axe AC de la colonne vertébrale CV, au-dessus du point de référence.

Dans une première étape illustrée en Figure 11, le chirurgien écarte les différents éléments tels que tissus, muscles, ligaments, etc. au moyen d'un écarteur (non illustré) et il insère par voie dorsale un pointeau de positionnement 6, formant un composant de l'ancillaire de pose, jusqu'à l'ancrer sur le point de référence.

Ce pointeau de positionnement 6 est une tige longiligne et rectiligne présentant une extrémité distale 61 en forme de pointe. Ce pointeau de positionnement 6 présente une section transversale rectangulaire, sans que cette forme ne soit limitative car le pointeau de positionnement 6 peut aussi avoir une section transversale circulaire, carrée, polygonale ou autre. L'extrémité distale 61 en pointe est ancrée sur le point de référence dans la partie latérale PL de vertèbre VE.

Ce pointeau de positionnement 6 présente également une extrémité proximale 62, opposée à l'extrémité distale 61, et le pointeau de positionnement 6 est suffisamment long pour que cette extrémité proximale 62 dépasse du dos du patient.

Dans une deuxième étape illustrée en Figure 12, le chirurgien insère un support primaire 7 en le faisant coulisser sur le pointeau de positionnement 6, comme schématisé par la flèche GU ; ce support primaire 7 étant un autre composant de l'ancillaire de pose.

Ce support primaire 7 se présente sous la forme d'un corps tubulaire et longiligne, présentant une partie distale 71 et une partie proximale 72 opposées. La partie distale 71 présente une face distale 710 tournée vers la vertèbre en situation, de laquelle fait saillie plusieurs picots 711 formant des reliefs d'ancrage configurés pour permettre un ancrage sur la partie latérale PL de vertèbre VE. La partie proximale 72 présente une face proximale 720 tournée vers l'extérieur, à l'opposé de la face distale 710.

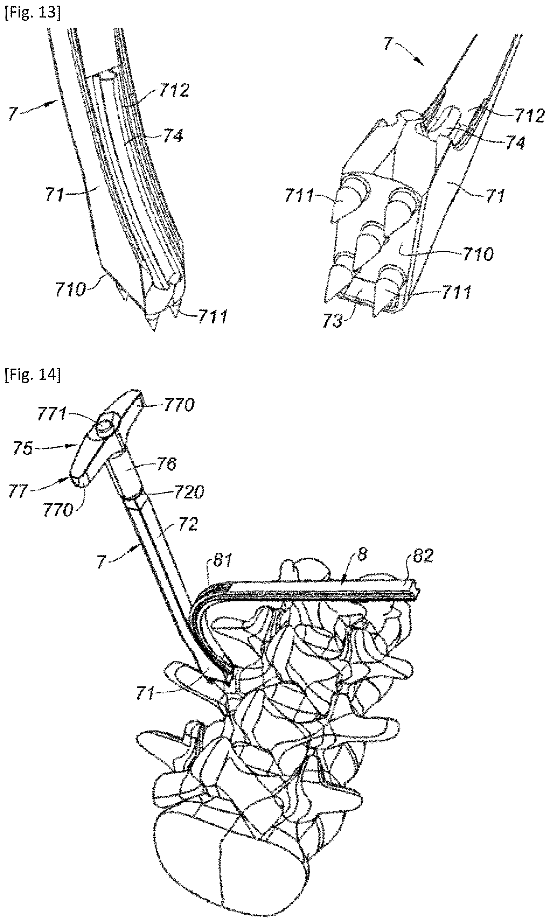

Le support primaire 7 présente un trou distal 73 (visible en Figure 13) débouchant dans sa face distale 710 et présente également un trou proximal 730 (visible en Figure 12) débouchant dans sa face proximale 720 ; ces deux trous 73, 730 pouvant être tous deux borgnes ou dans la continuité l'un de l'autre.

Ce support primaire 7 est plus long que le pointeau de positionnement 6 et le trou distal 73 est sensiblement plus long que le pointeau de positionnement 6. Ainsi, le pointeau de positionnement 6 est conformé pour guider longitudinalement en coulissement le support primaire 7 par insertion du pointeau de positionnement 6 à l'intérieur du trou distal 73.

Le support primaire 7 est introduit de l'extérieur au niveau de l'extrémité proximale 62, en s'insérant au niveau de la face proximale 720, puis le support primaire 7 coulisse le long du pointeau de positionnement 6 (comme schématisé par la flèche GU) jusqu'à ce que les picots 711 viennent au contact de la partie latérale PL de vertèbre VE.

La partie distale 71 du support primaire 7 s'élargit en direction de la face distale 710 et elle présente une face avant 712, tournée en direction des apophyses épineuses AE, ayant une forme incurvée et concave, de laquelle fait saillie un rail arqué 74 concave, ayant une section transversale en queue d'aronde.

Dans une troisième étape illustrée en Figure 12, le chirurgien accouple une poignée de maintien 75 sur la partie proximale 72 du support primaire 7, et plus précisément fixe la poignée de maintien 75 sur la face proximale 720 du support primaire 7.

Il est à noter que la poignée de maintien 75 pourrait, dans une variante préférentielle, être déjà accouplée au support primaire 7 durant la deuxième étape décrite ci-dessus, de sorte que la poignée de maintien 75 est déjà fixée au support primaire 7 avant l'insertion de ce support primaire 7 pour que le chirurgien puisse correctement maintenir et faire coulisser l'ensemble support primaire 7/poignée de maintien 75 sur le pointeau de positionnement 6.

Cette poignée de maintien 75 comprend un corps principal 76 prolongé d'un côté par une tête de préhension 77, et d'un autre côté par un pion d'emmanchement 78 propre à s'emmancher dans le trou proximal 730 du support primaire 7 pour un accouplement. La tête de préhension 77 est munie de deux ailes latérales 770 permettant une saisie manuelle de la poignée de maintien 7.

Avantageusement, la tête de préhension 77 est montée mobile en rotation à 90 degrés sur le corps principal 76, avec un système de verrouillage permettant de verrouiller la tête de préhension 77 dans une première position et dans une seconde position après avoir tourné de 90 degrés. Aussi, ce système de verrouillage comprend un bouton de déverrouillage 771 situé sur la tête de préhension 77, au milieu et dans l'alignement du corps principal 70 entre les deux ailes latérales 770, et qui permet de déverrouiller la rotation de la tête de préhension 77 pour passer de la première position vers la seconde position et inversement.

Le chirurgien va ensuite exercer, grâce à la poignée de maintien 75, une poussée sur le support primaire 7 afin de l'ancrer dans la partie latérale PL de vertèbre VE, en exerçant un effort de poussée, voire un effort d'impaction, de préférence répétée, sur la tête de préhension 77 pour enfoncer le support primaire 7.

Dans une quatrième étape illustrée en Figure 14, le chirurgien insère une broche d'implantation 8, autre composant de l'ancillaire de pose. Comme expliqué ultérieurement, une telle broche d'implantation 8 peut être pré-montée sur le support primaire 7 de sorte que le chirurgien insère l'ensemble support primaire 7/ poignée de maintien 75/ broche d'implantation 8 conjointement, en une seule étape. Cette broche d'implantation 8 présente une portion de terminaison distale 81 qui s'étend jusqu'à une extrémité distale 810 libre et en forme de pointe, et elle présente également une portion de terminaison proximale 82 qui prolonge la portion de terminaison distale 81 jusqu'à une extrémité proximale 820 libre.

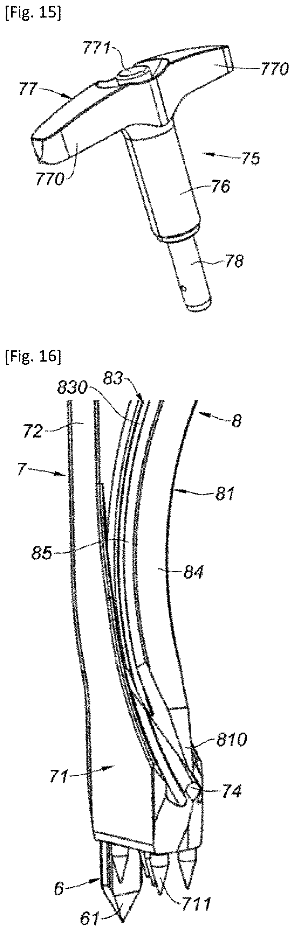

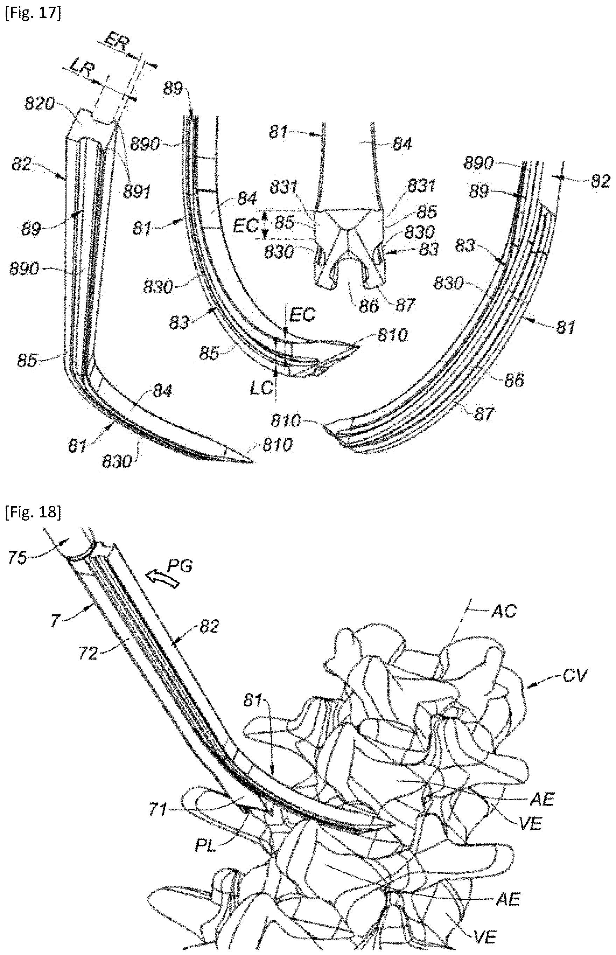

Comme visible sur les Figures 16 et 17, l'extrémité distale libre 810 en forme de pointe présente un angle de pointe inférieure à 90 degrés, et de préférence inférieure à 45 degrés.

La portion de terminaison distale 81 présente une forme incurvée et il est prévu, sur cette portion de terminaison distale 81, un rail de guidage 83 incurvé. Ce rail de guidage 83 est de forme complémentaire à la rainure antérieure 5 de l'implant 1, et présente le même rayon de courbure RC, pour permettre un guidage en coulissement de l'implant 1 ainsi que décrit ultérieurement. La portion de terminaison proximale 82 présente une forme rectiligne.

La courbure de la portion de terminaison distale 81 est telle que cette portion de terminaison distale 81 s'étend, par rapport à la portion de terminaison proximale 82, sur un secteur angulaire de l'ordre de 90 degrés, à plus ou moins 30 degrés. La portion de terminaison distale 81 est en particulier incurvée en arc de cercle selon le rayon de courbure RC donné sur un tel secteur angulaire de l'ordre de 90 degrés.

La broche d'implantation 8 présente une section transversale carrée ou rectangulaire, sur toute sa longueur partant de son extrémité distale 810 jusqu'à son extrémité proximale 820. La broche d'implantation 8 présente :
- une face antérieure 87 tournée vers le support primaire 7 en situation ;
- une face postérieure 84 opposée à la face antérieure 87 et tournée vers l'implant 1 lors de son insertion ;
- deux faces latérales 85 opposées, respectivement une face supérieure et une face inférieure.

Le rail de guidage 83 est réalisé sous la forme de deux encoches 830 ménagées dans les faces latérales 85 respectives au niveau de la portion de terminaison distale 81. Le rail de guidage 83 s'étend jusqu'à l'extrémité distale 810, autrement dit les encoches 830 s'étendent jusqu'à l'extrémité distale 810.

Ce rail de guidage 83 est incurvé en arc de cercle selon le rayon de courbure RC donné sur un secteur angulaire de l'ordre de 90 degrés, à plus ou moins 30 degrés.

Par ailleurs, le rail de guidage 83 est prolongé, à partir de la portion de terminaison distale 81, par un rail longitudinal 89 ménagé sur la portion de terminaison proximale 82 et s'étendant jusqu'à l'extrémité proximale 820. Ce rail longitudinal 89 est un rail rectiligne et il est formé de deux encoches 890 ménagées dans les faces latérales 85 respectives au niveau de la portion de proximale 82 et venant prolonger les encoches 830.

Ainsi, la broche d'implantation 8 présente successivement un rail de guidage 83 arqué le long de la portion de terminaison distale 81 également arquée, et un rail longitudinal 89 rectiligne le long de la portion de terminaison proximale 82 également rectiligne.

Par ailleurs, la portion de terminaison distale 81 présente une glissière arquée 86 ménagée sur la face antérieure 87. Cette glissière arquée 86 s'étend jusqu'à l'extrémité distale 810 sur un secteur angulaire de l'ordre de 90 degrés, à plus ou moins 30 degrés.

Cette glissière arquée 86 s'étend en particulier uniquement sur la portion de terminaison distale 81 et s'arrête au début de la portion de terminaison proximale 82. Dans une variante non illustrée, cette glissière arquée 86 s'étend sur la portion de terminaison distale 81 et se prolonge sur la portion de terminaison proximale 82 jusqu'à l'extrémité proximale 820, par exemple si au niveau de sa fabrication la broche d'implantation 8 est d'abord extrudée puis courbée.

Cette glissière arquée 86 est de forme complémentaire au rail arqué 74 prévu sur la face avant 712 de la partie distale 71 du support primaire 7. Ce rail arqué 74 et cette glissière arquée 86 forment respectivement un premier moyen de guidage en pivotement et un second moyen de guidage en pivotement propres à coopérer ensemble pour guider la broche d'implantation 8 en pivotement lors d'une insertion de sa portion de terminaison distale 81 entre les deux apophyses épineuses AE.

Aussi, dans la quatrième étape d'insertion de la broche d'implantation 8, comme visible en Figure 14, la broche d'implantation 8 est insérée avec sa portion de terminaison proximale 82 plus ou moins perpendiculaire au support primaire 7, et avec sa portion de terminaison distale 81 qui a son extrémité distale 810 qui pointe vers la face avant 712 de la partie distale 71 du support primaire 7, jusqu'à ce que la glissière arquée 86 s'engage avec le rail arqué 74.

Bien que non illustré, il est avantageux que la broche d'implantation 8 soit déjà pré-montée sur le support primaire 7 en position non-basculée (comme visible sur la Figure 14), de sorte que le chirurgien insère l'ensemble support primaire 7/ poignée de maintien 75/ broche d'implantation 8 conjointement (en une seule étape d'introduction), en faisant coulisser le tout sur le pointeau de positionnement 6 par une action sur la poignée de maintien 75.

Cependant, si le chirurgien doit écarter des tissus supplémentaires dans l'axe de l'abord latéral (axe AL de l'implant 1) au moyen par exemple d'un outil de raclage tel qu'une rugine, alors dans ce cas la broche d'implantation 8 ne serait pas montée directement sur le support primaire 7 pour faciliter l'écartement des tissus.

Dans une cinquième étape illustrée en Figure 18, le chirurgien fait pivoter (ou basculer) la broche d'implantation 8 relativement au support primaire 7 ancré et statique, comme schématisé par la flèche PG, jusqu'à ce que la portion de terminaison proximale 82 soit plaqué contre le support primaire 7 et que la portion de terminaison distale 81 s'étende en partie entre les deux apophyses épineuses AE.

Comme expliqué ci-dessus, ce pivotement de la broche d'implantation 8 est réalisé grâce à la coopération entre la glissière arquée 86 et le rail arqué 74. Le pivotement de la broche d'implantation 8 s'effectue sur un angle de l'ordre de 90 degrés.

Les formes complémentaires en queue d'aronde de la glissière arquée 86 et du rail arqué 74 permettent de maintenir le contact entre la glissière arquée 86 et le rail arqué 74 sans risque de décollement de la portion de terminaison distale 81 de la broche d'implantation 8 vis-à-vis du support primaire 7.

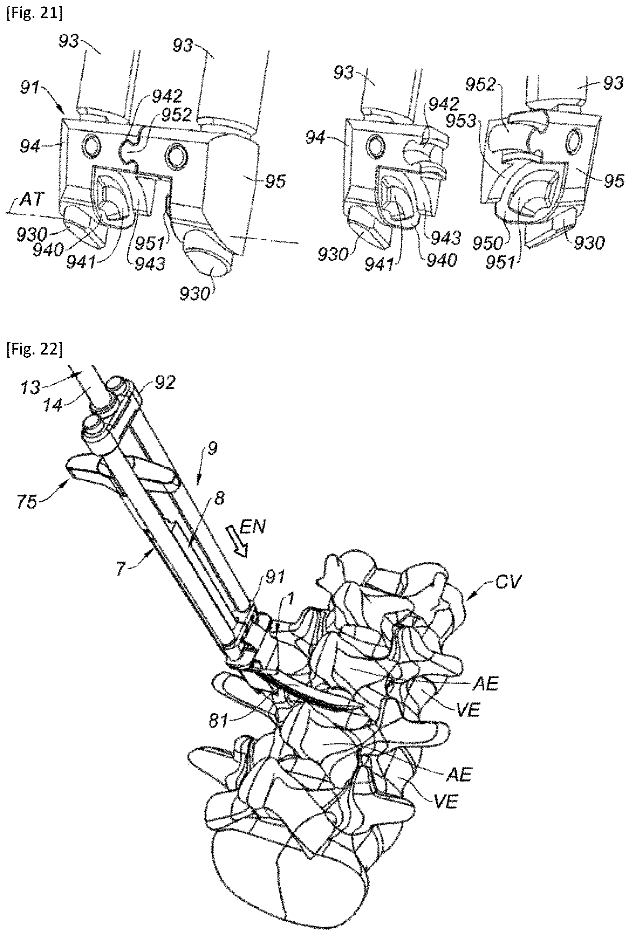

Dans une sixième étape illustrée en Figure 22, le chirurgien insère un ensemble comprenant un porte-ancillaire 9 et un impacteur 13 ; le porte-ancillaire 9 et l'impacteur 13 étant également des composants de l'ancillaire de pose.

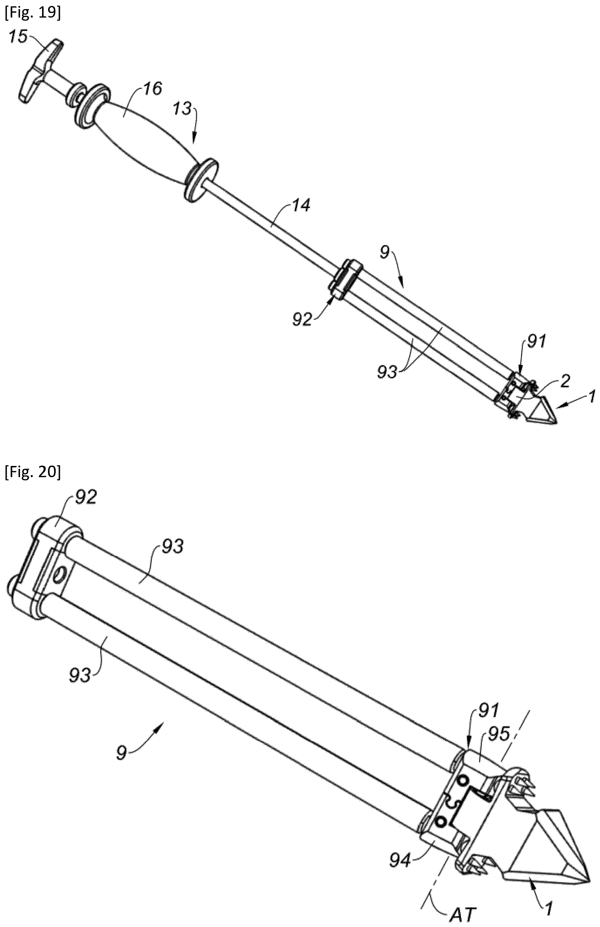

En référence aux Figures 19 et 20, le porte-ancillaire 9 présente une partie distale 91 et une partie proximale 92 opposées, reliées par deux tiges 93 parallèles.

La partie distale 91 est munie de moyens de support conformés pour supporter l'implant 1, où ces moyens de support comprennent deux éléments de support 94, 95 présentant des faces internes 940, 950 en vis-à-vis munies respectivement de protubérances 941, 951.

Ces protubérances 941, 951 sont conformés au moins partiellement en arc de cercle centré sur l'axe transversal AT et elles forment des moyens de pivotement complémentaires aux évidements 23 formés dans la partie arrière 2 de l'implant 1 pour autoriser un pivotement relatif de l'implant 1 sur ce porte-implant 9 selon l'axe transversal AT.

Comme visible en Figure 21, chaque protubérance 941, 951 se présente sous la forme d'une protubérance en arc de cercle selon un secteur angulaire inférieur à 180 degrés, comme par exemple et à titre non limitatif un secteur angulaire compris entre 20 et 175 degrés.

Par ailleurs, les éléments de support 94, 95 présentent également des faces arquées 943, 953 respectives, qui bordent les faces internes 940, 950 respectives, et qui sont conformés en arc de cercle centré sur l'axe transversal AT, où ces deux faces arquées 943, 953 sont prévues pour épouser la forme arrondie de l'extrémité arrière 20 de l'implant 1 pour pouvoir maintenir en place l'implant 1 et aussi pour pouvoir transmettre une poussée sur l'implant 1 lors de son insertion.

Ainsi, les protubérances 941, 951 portent l'implant 1 qui a sa partie arrière 2 coincé entre les faces internes 940, 950 avec les protubérances 941, 951 engagées dans les évidements 23, et qui a aussi son extrémité arrière 20 arrondie en appui contre les faces arquées 943, 953.

Les deux tiges 93 présentent des extrémités distales fixées sur les éléments de support 94, 95 respectifs. Par ailleurs, les deux éléments de support 94, 95 sont attachés l'un à l'autre de manière détachable et, à cet effet, ces éléments de support 94, 95 sont munis de moyens d'attachement amovible afin que les deux éléments de support 94, 95 soient configurables entre :
- une configuration attachée (à gauche sur la Figure 21) pour pouvoir supporter l'implant 1, et
- une configuration détachée (à droite sur la Figure 21) pour pouvoir l'implant 1 et ainsi ôter le porte-implant 9 ainsi que décrit ultérieurement.

Plus précisément et comme visible en Figure 21, les moyens d'attachement amovible sont formés d'une glissière supérieure 942 et d'un rail supérieur 952 qui sont prévus sur les éléments de support 94, 95 respectifs, ou inversement.

Cette glissière supérieure 942 et ce rail supérieur 952 sont tous deux arqués et de formes complémentaires, en queue d'aronde, et ils s'étendent entre une face antérieure et une face postérieure de la partie distale 91 ; la face antérieure étant tournée vers la broche d'implantation 8 en situation et la face postérieure, opposée à la face antérieure, étant tournée vers les apophyses épineuses AE en situation.

Cette glissière supérieure 942 et ce rail supérieur 952 forment des moyens de guidage en pivotement propres à coopérer ensemble pour guider en pivotement un élément de support 94 relativement à l'autre élément de support 95, selon l'axe transversal AT.

Dans la configuration attachée, le rail supérieur 952 est intégralement emboîté ou reçu à l'intérieur de la glissière supérieure 942 et les deux éléments de support 94, 95 sont attachés l'un à l'autre et peuvent supporter l'implant 1 sur leurs protubérances 941, 951.

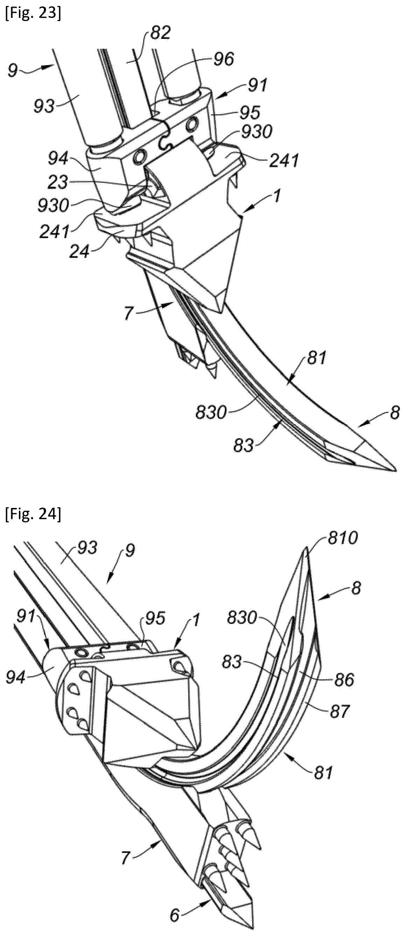

Par ailleurs, il est à noter que les extrémités distales des tiges 93 présentent des terminaisons 930 respectives qui dépassent des éléments de support 94, 95 respectifs, et ces terminaisons 930 ont pour fonction de venir en appui contre les faces arrière 241 des ailettes de blocage 24 respectives de l'implant 1 (comme visible en Figure 23), afin de maintenir en place l'implant 1 et aussi de pouvoir transmettre une poussée sur l'implant 1 lors de son insertion.

Pour passer de la configuration attachée à la configuration détachée, il suffit de faire pivoter l'un des éléments de support 94, 95 autour de l'axe transversal AT, ce qui ne gêne pas l'implant 1, jusqu'à ce que le rail supérieur 952 soit intégralement sorti de la glissière supérieure 942.

La face antérieure de la partie distale 91 du porte-implant 9 présente une rainure 96 pour recevoir partiellement la broche d'implantation 8, comme visible en Figure 23. Cette rainure 96 étant formée de deux demi-rainures en vis-à-vis ménagées dans les éléments de support 94, 95 respectifs.

Les deux tiges 93 présentent chacune une section transversale circulaire, sans que cette forme ne soit limitative car chaque tige 93 peut aussi avoir une section transversale rectangulaire, carrée, polygonale ou autre.

Les deux tiges 93 présentent des extrémités proximales, opposées à leurs extrémités distales, qui sont fixées de manière amovible sur la partie proximale 92. Cette partie proximale 92 se présente sous la forme d'un corps monobloc qui relie les extrémités proximales des deux tiges 93 entre elles, et cette partie proximale 92 peut être détaché des extrémités proximales des deux tiges 93 lorsque nécessaire, ainsi que décrit ultérieurement.

L'impacteur 13 comprend une tige d'impaction 14 munie d'une extrémité distale fixée sur la partie proximale 92, entre les extrémités proximales des deux tiges 93. Cette tige d'impaction 14 est également munie d'une extrémité distale, opposée à l'extrémité distale, et sur laquelle est fixée une poignée 15 formée deux ailes latérales permettant une saisie manuelle de l'impacteur 13. L'impacteur 13 comprend en outre un manchon d'impaction 16 monté coulissant autour de la tige d'impaction 14, entre la poignée 15 et la partie proximale 92 du porte-implant 9.

Le chirurgien peut ainsi exercer, grâce à l'impacteur 13, une poussée sur le porte-implant 9, et donc sur l'implant 1, afin de positionner l'implant 1 entre les deux apophyses épineuses AE, en exerçant un effort d'impaction, de préférence répétée, qui consiste à tenir avec une main la poignée 15 et à exercer avec l'autre main un mouvement coulissant du manchon d'impaction 16 afin qu'il vienne impacter sur la partie proximale 92 ; cet effort d'impaction étant ensuite transmis à travers les tiges 93 jusqu'aux éléments de support 94, 95, et enfin à travers les terminaisons 930 et les faces arquées 943, 953 jusqu'à l'implant 1.

Au début de la sixième étape, l'implant 1 est glissé sur le rail longitudinal 89 de la broche d'implantation 8, le long de sa face postérieure 84, au niveau de son extrémité proximale 820, en engageant sa rainure antérieure 5 par sa bouche avant 55 sur le rail longitudinal 89, et avec son extrémité avant 40 qui pointe en direction de la partie latérale PL de vertèbre VE.

Plus précisément, les lèvres de retenue 53 de la rainure antérieure 5 sont engagées à l'intérieur des encoches 890 ménagées dans les faces latérales 85 respectives de la broche d'implantation 8. Les lèvres de retenue 53 permettent de maintenir le contact entre la rainure antérieure 5 et le rail longitudinal 89 sans risque de décollement de l'implant 1 vis-à-vis de la broche d'implantation 8.

Il est à noter que, du fait de la forme arquée de la rainure antérieure 5 et de ses lèvres de retenue 53, le rail longitudinal 89 est plus large que le rail de guidage 83 arqué. Autrement dit, et comme visible en Figure 17, les encoches 890 du rail longitudinal 89 ont une largeur LR, et elles sont prolongées par les encoches 830 qui ont une largeur LC inférieur à LR.

Plus précisément, et comme visible sur la Figure 17, chaque encoche 890 du rail longitudinal 89 est délimité au niveau de la face postérieure 84 par un bord d'encoche 891 présentant une épaisseur ER. De même, et comme visible sur la Figure 17, chaque encoche 830 du rail de guidage 83 arqué est délimité au niveau de la face postérieure 84 par un bord d'encoche 831 présentant une épaisseur EC, où EC est supérieure à ER, et où LR + ER = LC + EC. Ainsi, chaque bord d'encoche 891 est prolongé par le bord d'encoche 831 correspondant, avec un élargissement de l'un vers l'autre.

Il est à noter que l'épaisseur ER est sensiblement équivalente à la hauteur intermédiaire HI de la rainure antérieure 5 (voir Figure 5), et la largeur LR est sensiblement équivalente à la hauteur globale HG des lèvres de retenue 53 de la rainure antérieure 5 (voir Figure 5), de sorte que l'implant 1 peut coulisser selon une trajectoire rectiligne le long du rail longitudinal 89, avec les lèvres de retenue 53 de sa rainure antérieure 5 qui coulissent dans les encoches 890 ; les bords d'encoche 891 glissant quant à eux dans l'interstice longitudinal définit par cette hauteur intermédiaire HI.

Par ailleurs, l'épaisseur EC est sensiblement équivalente à la hauteur de rainure HR de la rainure antérieure 5 (voir Figure 5), et la largeur LC est sensiblement équivalente à la hauteur de lèvre HL des lèvres de retenue 53 de la rainure antérieure 5 (voir Figure 5), de sorte que l'implant 1 peut coulisser selon une trajectoire arquée (centrée sur le centre de courbure de la face antérieure 11 de l'implant 1 qui se confond avec le centre de courbure du rail de guidage 83), avec les lèvres de retenue 53 de sa rainure antérieure 5 qui coulissent dans les encoches 830.

Lors de la sixième étape, le chirurgien pousse donc sur le porte-implant 9, au moyen de l'impacteur 13, pour faire descendre le porte-implant 9 et l'implant 1 le long du rail longitudinal 89 et donc de la portion de terminaison proximale 82 de la broche d'implantation 8, comme schématisé par la flèche EN sur la Figure 22, jusqu'à ce que la rainure antérieure 5 atteigne le rail de guidage 83 arqué.

Il est à noter que le chirurgien a préalablement fait pivoter de 90 degrés la tête de préhension 77 de la poignée de maintien 75, après avoir enfoncé le bouton de déverrouillage 771, pour que la tête de préhension 77 puisse passer entre les deux tiges 93, comme visible en Figure 22.

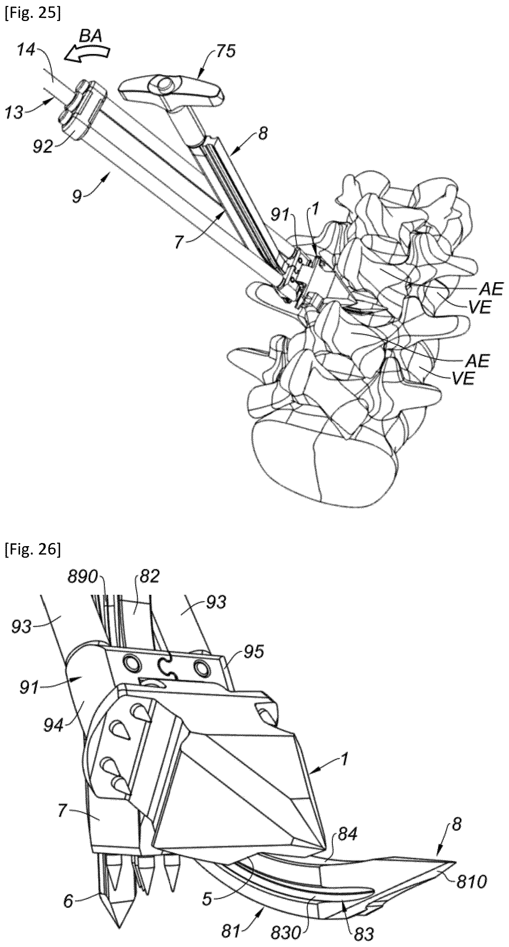

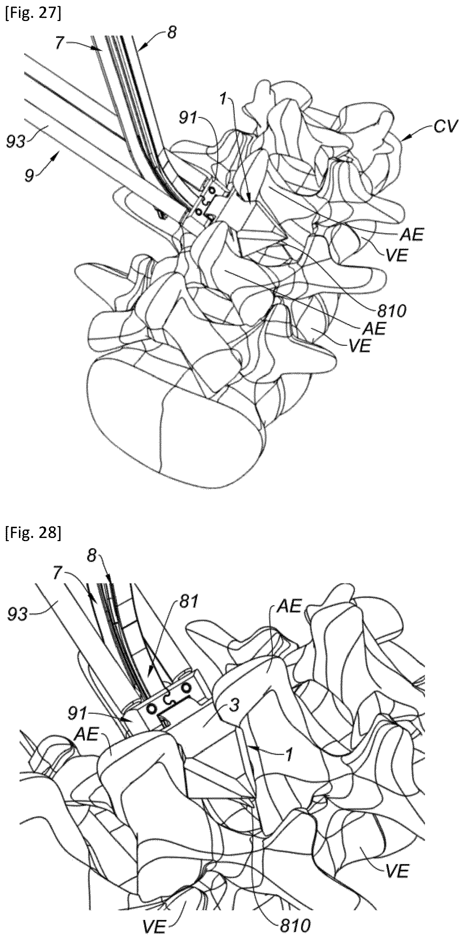

Dans une septième étape illustrée aux Figures 25 à 28, le chirurgien continue de pousser sur le porte-implant 9, au moyen de l'impacteur 13, pour faire coulisser l'implant 1 le long du rail de guidage 83 arqué (en regard de la face postérieure 84) de la portion de terminaison distale 81, donc suivant une trajectoire curviligne qui amène l'implant 1 à pivoter selon un angle de l'ordre de 90 degrés et à se diriger en direction de l'espace interépineux entre les deux apophyses épineuses AE, jusqu'à ce que l'implant 1 atteigne une position finale entre les deux apophyses épineuses AE, comme visible aux Figures 27 et 28.

Dans cette septième étape, les lèvres de retenue 53 de la rainure antérieure 5 sont engagées à l'intérieur des encoches 830, après être passées par les encoches 890. Ces lèvres de retenue 53 permettent de maintenir le contact entre la rainure antérieure 5 et le rail de guidage 83 sans risque de décollement de l'implant 1 vis-à-vis de la broche d'implantation 8.

Lors de ce déplacement curviligne de l'implant 1, cet implant 1 pivote relativement au porte-implant 9, grâce aux protubérances 941, 951 qui coopèrent avec les évidements 23, et passe d'une orientation initiale substantiellement parallèle au support primaire 7 à une orientation finale substantiellement perpendiculaire au support primaire 7.

Ainsi, le porte-implant 9 pivote (ou bascule) concomitamment au coulissement de l'implant 1, comme schématisé par la flèche BA sur la Figure 25, avec la partie proximale 92 qui passe par-dessus la poignée de maintien 75. Lors de cette insertion, la forme fuselée de la partie avant 4 de l'implant 1 permet d'écarter les deux apophyses épineuses AE, pour amener sa partie centrale 3 entre les deux apophyses épineuses AE.

Une fois l'implant 1 en place, le chirurgien peut effectuer une radio de contrôle. Au besoin il peut retirer l'implant 1 en tirant sur la tête d'impaction 15 pour faire remonter le porte-implant 9 et donc l'implant 1. Si l'implant est correctement placé, le chirurgien passe à la huitième étape décrite ci-dessous.

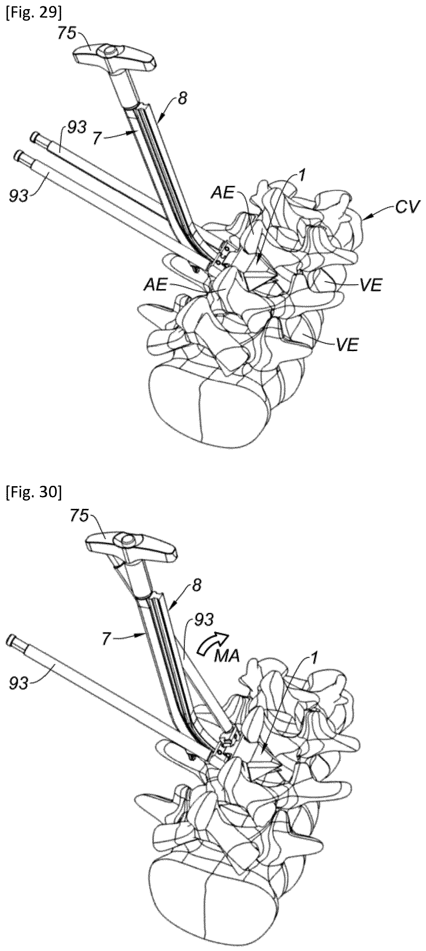

Dans une huitième étape illustrée en Figure 29, le chirurgien retire l'impacteur 13 vis-à-vis du porte-implant 9, et pour ce faire il détache la partie proximale 92 des tiges 93 et il tire sur la poignée 15 pour retirer cet impacteur 13.

Dans une neuvième étape illustrée en Figure 30, le chirurgien déverrouille le porte-implant 9 en faisant passer ses deux éléments de support 94, 95 en configuration détachée.

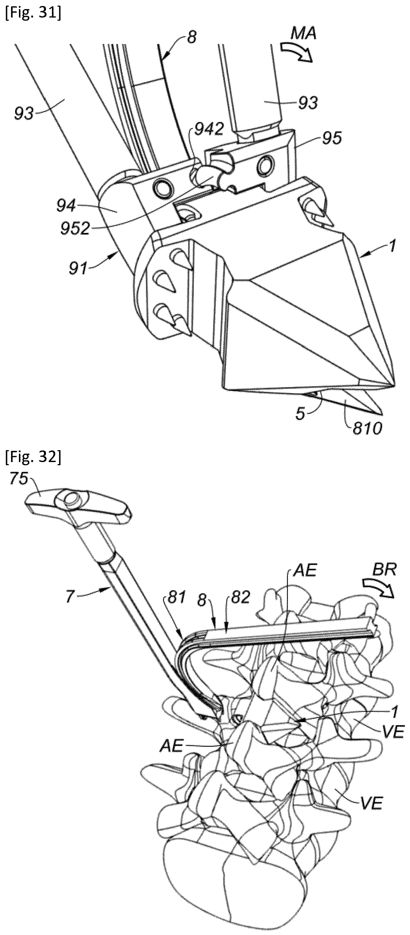

Pour ce faire, le chirurgien agit sur les tiges 93 pour faire pivoter un élément de support 94 relativement à l'autre élément de support 95, comme schématisé par la flèche MA sur les Figures 30 et 31, jusqu'à ce que le rail supérieur 952 soit intégralement sorti de la glissière supérieure 942, comme visible en Figure 31.

Ainsi, le chirurgien poursuit en retirant complètement le porte-implant 9 qui est désolidarisé de l'implant 1, en tirant sur une tige 93 qui emporte avec elle l'élément de support 94 qui lui est attaché, et ensuite en tirant sur l'autre tige 93 qui emporte avec elle l'autre élément de support 95 qui lui est attaché.

Dans une dixième étape illustrée en Figure 32, le chirurgien retire la broche d'implantation 8, et pour ce faire il fait pivoter (ou basculer) la broche d'implantation 8 relativement au support primaire 7 ancré et statique, comme schématisé par la flèche BR, pour éloigner la portion de terminaison proximale 82 du support primaire 7 et faire sortir la portion de terminaison distale 81 de la rainure antérieure 5 (par sa bouche arrière 54) de l'implant 1.

Ce pivotement de la broche d'implantation 8 s'effectue sur un angle de l'ordre de 90 degrés, selon une rotation inverse à celle effectuée lors de sa mise en place sur le support primaire 7.

Ainsi, le chirurgien poursuit en retirant complètement la broche d'implantation 8, puis il tire sur le support primaire 7, au moyen de la tête de préhension 77 de la poignée de maintien 75, pour retirer le support primaire 7, et enfin le chirurgien retire le pointeau de positionnement 6.

A ce stade, seul l'implant 1 est en place entre les apophyses épineuses AE, comme illustré sur les Figures 8 à 10, et le chirurgien peut terminer l'intervention, en particulier en retirant l'écarteur et en refermant l'incision.

## Revendications

1. Implant vertébral interépineux (1) comportant un corps d'implant (10) s'étendant selon un axe longitudinal (AL) et comprenant successivement le long dudit axe longitudinal (AL) :
- une partie arrière (2) présentant une extrémité arrière (20),
- une partie centrale (3) conformée pour s'étendre entre deux apophyses épineuses (AE) de deux vertèbres (VE) adjacentes, ladite partie centrale (3) prolongeant la partie arrière (2), et
- une partie avant (4) prolongeant la partie centrale (3), de manière opposée à la partie arrière (2), en s'effilant jusqu'à une extrémité avant (40) de forme fuselée, dans lequel ledit corps d'implant (10) présente :
- une face antérieure (11) destinée à être orientée vers les vertèbres (VE),
- un canal de guidage ménagé dans le corps d'implant (10) de son extrémité arrière (20) jusqu'à son extrémité avant (40), ledit canal de guidage étant conformé pour recevoir au moins une portion d'une broche d'implantation (8) d'un ancillaire de pose ;
ledit implant vertébral interépineux (1) étant **caractérisé en ce que** le canal de guidage s'étend depuis l'extrémité arrière (20) jusqu'à l'extrémité avant (40) suivant une direction curviligne, ledit canal de guidage étant ménagé dans la face antérieure (11) de manière à ce que ledit canal de guidage forme une rainure antérieure (5) de forme curviligne et débouchant extérieurement dans ladite face antérieure (11) du corps d'implant (10).

2. Implant vertébral interépineux (1) selon la revendication 1, dans lequel la rainure antérieure (5) est ménagée de manière continue dans la face antérieure (11) du corps d'implant (10) depuis l'extrémité arrière (20) jusqu'à l'extrémité avant (40), de manière à déboucher continuellement dans la face antérieure (11).

3. Implant vertébral interépineux (1) selon les revendications 1 ou 2, dans lequel le corps d'implant (10) est non articulé, de sorte que, dans une position de repos comme dans une position implantée, la rainure antérieure (5) est de forme curviligne selon une même courbure.

4. Implant vertébral interépineux (1) selon l'une quelconque des revendications précédentes, dans lequel la rainure antérieure (5) est de forme convexe selon un rayon de courbure (RC) donné, avec un centre de courbure situé en regard d'une face postérieure (12) du corps d'implant (10), opposée à la face antérieure (11).

5. Implant vertébral interépineux (1) selon la revendication 4, dans lequel la rainure antérieure (5) est délimitée intérieurement par une paroi de fond (50) décalée en profondeur par rapport à la face antérieure (11), ladite paroi de fond (50) étant de forme convexe selon un rayon de courbure donné.

6. Implant vertébral interépineux (1) selon la revendication 5, dans lequel la rainure antérieure (5) est délimitée extérieurement par des lèvres de retenue (53) de forme convexe selon le rayon de courbure (RC), lesdites lèvres de retenue (53) étant disposées en regard de la paroi de fond (50) et s'étendant l'une en face de l'autre pour délimiter un espace rétréci comparativement à l'intérieur de la rainure antérieure (5).

7. Implant vertébral interépineux (1) selon l'une quelconque des revendications 1 à 6, dans lequel la partie arrière (2) présente des moyens de pivotement (23) selon un axe transversal (AT) s'étendant transversalement par rapport à l'axe longitudinal (AL) pour permettre un pivotement dudit corps d'implant (10) relativement à des moyens de pivotement complémentaires (941, 951) prévus sur un porte-implant (9) de l'ancillaire de pose.

8. Implant vertébral interépineux (1) selon l'une quelconque des revendications 1 à 7, dans lequel la partie arrière (2) comprend, à une interface avec la partie centrale (3), au moins une ailette de blocage (24) faisant saillie sur un côté inférieur de la partie arrière (2) ou sur un côté supérieur de la partie arrière (2), opposé au côté inférieur.

9. Ancillaire de pose, prévu pour un guidage et un positionnement d'un implant vertébral interépineux (1) selon l'une quelconque des revendications 1 à 8 entre deux apophyses épineuses (AE) de deux vertèbres (VE) adjacentes, ledit ancillaire de pose comprenant :
- un porte-implant (9) présentant une partie distale (91) et une partie proximale (92) opposées, ladite partie distale (91) étant munie de moyens de support (94, 95) conformés pour supporter ledit implant vertébral interépineux (1) ;
- une broche d'implantation (8) présentant une portion de terminaison distale (81) qui s'étend jusqu'à une extrémité distale (810) libre et en forme de pointe, ladite portion de terminaison distale (81) présentant une forme incurvée et sur laquelle est prévu un rail de guidage (83) incurvé et de forme complémentaire à une rainure antérieure (5) de l'implant vertébral interépineux (1) pour permettre un guidage en coulissement dudit implant vertébral interépineux (1) le long d'une face postérieure (84) de ladite portion de terminaison distale (81) par engagement du rail de guidage (83) à l'intérieur de la rainure antérieure (5), ledit rail de guidage (83) s'étendant jusqu'à l'extrémité distale (810) libre.

10. Ancillaire de pose selon la revendication 9, dans lequel la portion de terminaison distale (81) de la broche d'implantation (8) est incurvée en arc de cercle sur un secteur angulaire compris entre 60 et 120 degrés.

11. Ancillaire de pose selon la revendication 9 ou 10, dans lequel la broche d'implantation (8) comprend une portion de terminaison proximale (82) qui prolonge la portion de terminaison distale (81) jusqu'à une extrémité proximale (820) libre, et le rail de guidage (83) est prolongé, à partir de la portion de terminaison distale (81), par un rail longitudinal (89) ménagé sur la portion de terminaison proximale (82) jusqu'à l'extrémité proximale (820).

12. Ancillaire de pose selon la revendication 11, dans lequel l'un au moins de la portion de terminaison proximale (82) et du rail longitudinal (89) est rectiligne.

13. Ancillaire de pose selon l'une quelconque des revendications 9 à 12, dans lequel la portion de terminaison distale (81) de la broche d'implantation (8) présente deux faces latérales (85) opposées, respectivement une face supérieure et une face inférieure, et le rail de guidage (83) est ménagé sous la forme de deux encoches (830) ménagées dans lesdites faces latérales (85) respectives.

14. Ancillaire de pose selon l'une quelconque des revendications 9 à 13, dans lequel la portion de terminaison distale (81) de la broche d'implantation (8) présente une face antérieure (87), opposée à la face postérieure (84) le long de laquelle coulisse l'implant (1), et sur laquelle est prévu un premier moyen de guidage en pivotement (86),
et ledit ancillaire de pose comprend un support primaire (7) présentant une partie distale (71) sur laquelle sont prévus au moins un relief d'ancrage (711) pour un ancrage, et un second moyen de guidage en pivotement (74) propre à coopérer avec le premier moyen de guidage en pivotement (86) pour guider ladite broche d'implantation (8) en pivotement lors d'une insertion de sa portion de terminaison distale (81).

15. Ancillaire de pose selon la revendication 14, dans lequel le support primaire (7) présente un trou distal (73) débouchant dans une face distale (710) de la partie distale (71) du support primaire (7), et l'ancillaire de pose comprend une pointeau de positionnement (6) munie d'une extrémité distale (61) en pointe prévue pour un ancrage dans une vertèbre (VE), ledit pointeau de positionnement (6) étant conformée pour guider longitudinalement en coulissement le support primaire (7) par insertion du pointeau de positionnement (6) à l'intérieur du trou distal (73).

16. Ancillaire de pose selon l'une quelconque des revendications 9 à 15, dans lequel les moyens de support (94, 95) prévus sur la partie distale (91) du porte-implant (9) comprennent des moyens de pivotement complémentaires (941, 951) conformés pour coopérer avec des moyens de pivotement (23) d'une partie arrière (2) de l'implant (1) afin d'autoriser un pivotement dudit implant (1) selon un axe transversal (AT).

17. Ancillaire de pose selon l'une quelconque des revendications 9 à 16, dans lequel l'ancillaire de pose comprend un impacteur (13) monté de manière amovible sur la partie proximale (92) du porte-implant (9).

## Patentansprüche

1. Interspinöses Wirbelkörperimplantat (1) mit einem Implantatkörper (10), der sich entlang einer Längsachse (AL) erstreckt und nacheinander entlang dieser Längsachse (AL) Folgendes umfasst:
- ein hinteres Teil (2) mit einem hinteren Ende (20),
- einen zentralen Teil (3), der so geformt ist, dass er sich zwischen zwei Dornfortsätzen (AE) zweier benachbarter Wirbel (VE) erstreckt, wobei der zentrale Teil (3) den hinteren Teil (2) verlängert, und
- einen vorderen Teil (4), der den zentralen Teil (3) gegenüber dem hinteren Teil (2) verlängert und sich bis zu einem konisch zulaufenden vorderen Ende (40) verdünnt, wobei der Implantatkörper (10) Folgendes aufweist:
- eine Vorderseite (11), die dazu bestimmt ist, den Wirbeln (VE) zugewandt zu sein,
- einen Führungskanal, der im Implantatkörper (10) von seinem hinteren Ende (20) bis zu seinem vorderen Ende (40) vorgesehen ist, wobei der Führungskanal so ausgebildet ist, dass er mindestens einen Teil eines Implantationsstifts (8) einer Montagehilfe aufnimmt;
wobei das interspinöse Wirbelkörperimplantat (1) **dadurch gekennzeichnet ist, dass** sich der Führungskanal vom hinteren Ende (20) bis zum vorderen Ende (40) in einer kurvenförmigen Richtung erstreckt, wobei der Führungskanal in der Vorderseite (11) so ausgebildet ist, dass der Führungskanal eine kurvenförmige Vordernut (5) bildet, die nach außen in die Vorderseite (11) des Implantatkörpers (10) mündet.

2. Interspinöses Wirbelkörperimplantat (1) nach Anspruch 1, wobei die Vordernut (5) von dem hinteren Ende (20) bis zum vorderen Ende (40) durchgehend in die Vorderseite (11) des Implantatkörpers (10) eingebracht ist, so dass sie durchgehend in die Vorderseite (11) mündet.

3. Interspinöses Wirbelkörperimplantat (1) nach Anspruch 1 oder 2, wobei der Implantatkörper (10) ungelenkt ist, so dass in einer Ruheposition wie in einer implantierten Position die Vordernut (5) kurvenförmig in derselben Krümmung ausgebildet ist.

4. Interspinöses Wirbelkörperimplantat (1) nach einem der vorhergehenden Ansprüche, wobei die Vordernut (5) nach einem bestimmten Krümmungsradius (RC) konvex geformt ist, mit einem Krümmungszentrum gegenüber einer Hinterseite (12) des Implantatkörpers (10), die der Vorderseite (11) gegenüberliegt.

5. Interspinöses Wirbelkörperimplantat (1) nach Anspruch 4, wobei die Vordernut (5) innen durch eine von der Vorderseite (11) in der Tiefe versetzte Bodenwand (50) begrenzt ist, wobei die Bodenwand (50) nach einem bestimmten Krümmungsradius konvex geformt ist.

6. Interspinöses Wirbelkörperimplantat (1) nach Anspruch 5, wobei die Vordernut (5) nach außen durch konvex geformte Haltelippen (53) gemäß dem Krümmungsradius (RC) begrenzt ist, wobei die Haltelippen (53) gegenüber der Bodenwand (50) angeordnet sind und sich gegenüberliegend erstrecken, um einen vergleichsweise verengten Raum innerhalb der Vordernut (5) zu begrenzen.

7. Interspinöses Wirbelkörperimplantat (1) nach einem der Ansprüche 1 bis 6, wobei der hintere Teil (2) Schwenkmittel (23) entlang einer Querachse (AT) aufweist, die sich quer zur Längsachse (AL) erstrecken, um ein Schwenken des Implantatkörpers (10) in Bezug auf ergänzende Schwenkmittel (941, 951) zu ermöglichen, die an einem Implantathalter (9) der Montagehilfe vorgesehen sind.

8. Interspinöses Wirbelkörperimplantat (1) nach einem der Ansprüche 1 bis 7, wobei der hintere Teil (2) an einer Schnittstelle mit dem zentralen Teil (3) mindestens eine Sperrrippe (24) umfasst, die an einer Unterseite des hinteren Teils (2) oder an einer Oberseite des hinteren Teils (2), gegenüber der Unterseite, hervorsteht.

9. Montagehilfe, vorgesehen zur Führung und Positionierung eines interspinösen Wirbelkörperimplantats (1) nach einem der Ansprüche 1 bis 8 zwischen zwei Dornfortsätzen (AE) zweier benachbarter Wirbel (VE), wobei die Montagehilfe Folgendes umfasst:
- einen Implantathalter (9), der einen gegenüberliegenden distalen Teil (91) und einen proximalen Teil (92) aufweist, wobei der distale Teil (91) mit Stützmitteln (94, 95) versehen ist, die so geformt sind, dass sie das interspinöse Wirbelkörperimplantat (1) stützen;
- einen Implantationsstift (8) mit einem distalen Endabschnitt (81), der sich bis zu einem freien und spitzenförmigen distalen Ende (810) erstreckt, wobei der distale Endabschnitt (81) eine gebogene Form aufweist und an dem eine gebogene und komplementär zu einer Vordernut (5) des interspinösen Wirbelkörperimplantats (1) geformte Führungsschiene (83) vorgesehen ist, um eine Gleitführung des interspinösen Wirbelkörperimplantats (1) entlang einer Hinterseite (84) zu ermöglichen des distalen Endabschnitts (81) durch Einrasten der Führungsschiene (83) in die Vordernut (5), wobei sich die Führungsschiene (83) bis zu dem freien distalen Ende (810) erstreckt.

10. Montagehilfe nach Anspruch 9, wobei der distale Endabschnitt (81) des Implantationsstifts (8) in einem Kreisbogen über einen Winkelsektor zwischen 60 und 120 Grad gebogen ist.

11. Montagehilfe nach Anspruch 9 oder 10, wobei der Implantationsstift (8) einen proximalen Endabschnitt (82) umfasst, der den distalen Endabschnitt (81) bis zu einem freien proximalen Ende (820) verlängert, und die Führungsschiene (83) von dem distalen Endabschnitt (81) durch eine Längsschiene (89) verlängert ist, die am proximalen Endabschnitt (82) bis zum proximalen Ende (820) vorgesehen ist.

12. Montagehilfe nach Anspruch 11, wobei mindestens einer des proximalen Endbschnitts (82) und der Längsschiene (89) gerade ist.

13. Montagehilfe nach einem der Ansprüche 9 bis 12, wobei der distale Endabschnitt (81) des Implantationsstifts (8) zwei gegenüberliegende Seitenflächen (85) aufweist, jeweils eine Oberseite und eine Unterseite, und die Führungsschiene (83) in Form von zwei in den jeweiligen Seitenflächen (85) vorgesehenen Kerben (830) ausgebildet ist.

14. Montagehilfe nach einem der Ansprüche 9 bis 13, wobei der distale Endabschnitt (81) des Implantationsstifts (8) eine Vorderseite (87) aufweist, die der Hinterseite (84) gegenüberliegt, entlang der das Implantat (1) gleitet, und an der ein erstes Schwenkführungsmittel (86) vorgesehen ist, und die Montagehilfe eine Primärstütze (7) umfasst, der einen distalen Teil (71) aufweist, an dem zumindest eine Verankerungserhebung (711) für eine Verankerung vorgesehen ist, und ein zweites Schwenkführungsmittel (74), das mit dem ersten Schwenkführungsmittel (86) zusammenarbeiten kann, um den Implantationsstift (8) beim Einführen seines distalen Endabschnitt (81) in Schwenkrichtung zu führen.

15. Montagehilfe nach Anspruch 14, wobei die Primärstütze (7) ein distales Loch (73) aufweist, das in eine distale Seite (710) des distalen Teils (71) der Primärstütze (7) mündet, und die Montagehilfe eine Positionierungsnadel (6) umfasst, die mit einem distalen Ende (61) an einer Spitze versehen ist, die für die Verankerung in einem Wirbel (VE) vorgesehen ist, wobei die Positionierungsnadel (6) so geformt ist, dass sie die Primärstütze (7) längs verschieblich durch Einführen der Positionierungsnadel (6) in das distale Loch (73) führt.

16. Montagehilfe nach einem der Ansprüche 9 bis 15, wobei die am distalen Teil (91) des Implantathalters (9) vorgesehenen Stützmittel (94, 95) ergänzende Schwenkmittel (941, 951) umfassen, die so gestaltet sind, dass sie mit Schwenkmitteln (23) eines hinteren Teils (2) des Implantats (1) zusammenarbeiten, um ein Schwenken des Implantats (1) um eine Querachse (AT) zu ermöglichen.

17. Montagehilfe nach einem der Ansprüche 9 bis 16, wobei die Montagehilfe einen am proximalen Teil (92) des Implantathalters (9) beweglich montierten Einschläger (13) umfasst.

## Claims

1. An interspinous vertebral implant (1) including an implant body (10) extending along a longitudinal axis (AL) and comprising successively along said longitudinal axis (AL):
- a rear portion (2) having a rear end (20),
- a central portion (3) shaped so as to extend between two spinous processes (AE) of two adjacent vertebrae (VE), said central portion (3) extending the rear portion (2), and
- a front portion (4) extending the central portion (3), opposite to the rear portion (2), while tapering up to a front end (40) of streamlined shape,
wherein said implant body (10) has:
- an anterior face (11) intended to be directed towards the vertebrae (VE),
- a guide channel formed in the implant body (10) from its rear end (20) up to its front end (40), said guide channel being shaped so as to receive at least one portion of an implantation spindle (8) of an implantation ancillary,
said interspinous vertebral implant (1) being **characterized in that** the guide channel extends from the rear end (20) up to the front end (40) along a curvilinear direction, said guide channel being formed in the anterior face (11) so that said guide channel forms an anterior groove (5) of curvilinear shape and opening outside into said anterior face (11) of the implant body (10).

2. The interspinous vertebral implant (1) according to claim 1, wherein the anterior groove (5) is formed continuously in the anterior face (11) of the implant body (10) from the rear end (20) up to the front end (40), so as to continuously open into the anterior face (11).

3. The interspinous vertebral implant (1) according to claims 1 or 2, wherein the implant body (10) is not articulated, so that, in a rest position as well as in an implanted position, the anterior groove (5) is curvilinear shaped along the same curvature.

4. The interspinous vertebral implant (1) according to any one of the preceding claims, wherein the anterior groove (5) has a convex shape having a given radius of curvature (RC), with a center of curvature located facing a posterior face (12) of the implant body (10), opposite to the anterior face (11).

5. The interspinous vertebral implant (1) according to claim 4, wherein the anterior groove (5) is internally delimited by a bottom wall (50) offset in depth with respect to the anterior face (11), said bottom wall (50) having a convex shape having a given radius of curvature.

6. The interspinous vertebral implant (1) according to claim 5, wherein the anterior groove (5) is externally delimited by retaining lips (53) with a convex shape having the radius of curvature (RC), said retaining lips (53) being disposed facing the bottom wall (50) and extending facing each other so as to delimit a narrow space in comparison with the interior of the anterior groove (5).

7. The interspinous vertebral implant (1) according to any one of claims 1 to 6, wherein the rear portion (2) has pivot means (23) around a transverse axis (AT) extending transversely with respect to the longitudinal axis (AL) to enable a pivoting of said implant body (10) relative to complementary pivot means (941, 951) provided on an implant-holder (9) of the implantation ancillary.

8. The interspinous vertebral implant (1) according to any one of claims 1 to 7, wherein the rear portion (2) comprises, at an interface with the central portion (3), at least one blocking plate (24) projecting on a lower side of the rear portion (2) or on an upper side of the rear portion (2), opposite to the lower side.

9. An implantation ancillary, intended for guidance and positioning of an interspinous vertebral implant (1) according to any one of claims 1 to 8 between two spinous processes (AE) of two adjacent vertebrae (VE), said implantation ancillary comprising:
- an implant-holder (9) having a distal portion (91) and a proximal portion (92) opposite to each other, said distal portion (91) being provided with support means (94, 95) shaped so as to support said interspinous vertebral implant (1);
- an implantation spindle (8) having a distal end portion (81) which extends up to a free distal end (810) of tip shape, said distal end portion (81) having a curved shape and on which is provided a curved guide rail (83) with a shape complementary with an anterior groove (5) of the interspinous vertebral implant (1) to enable a sliding guidance of said interspinous vertebral implant (1) along a posterior face (84) of said distal end portion (81) by fitting of the guide rail (83) inside the anterior groove (5), said guide rail (83) extending up to the free distal end (810).

10. The implantation ancillary according to claim 9, wherein the distal end portion (81) of the implantation spindle (8) is curved in a circular arc over an angular sector comprised between 60 and 120 degrees.

11. The implantation ancillary according to claim 9 or 10, wherein the implantation spindle (8) comprises a proximal end portion (82) which extends the distal end portion (81) up to a free proximal end (820), and the guide rail (83) is prolonged, from the distal end portion (81), by a longitudinal rail (89) formed on the proximal end portion (82) up to the free proximal end (820).

12. The implantation ancillary according to claim 11, wherein at least one amongst the proximal end portion (82) and the longitudinal rail (89) is rectilinear.

13. The implantation ancillary according to any one of claims 9 to 12, wherein the distal end portion (81) of the implantation spindle (8) has two opposite lateral faces (85), respectively an upper face and a lower face, and the guide rail (83) is formed in the form of two notches (830) formed in said respective lateral faces (85).

14. The implantation ancillary according to any one of claims 9 to 13, wherein the distal end portion (81) of the implantation spindle (8) has an anterior face (87), opposite to the posterior face (84) along which the interspinous vertebral implant (1) slides, and on which is provided a first pivot guide means (86),
and said implantation ancillary comprises a primary support (7) having a distal portion (71) on which are provided at least one anchoring relief (711) for anchorage, and a second pivot guide means (74) adapted to cooperate with the first pivot guide means (86) to pivotably guide said implantation spindle (8) during an insertion of its distal end portion (81).

15. The implantation ancillary according to claim 14, wherein the primary support (7) has a distal hole (73) opening into a distal face (710) of the distal portion (71) of the primary support (7), and the implantation ancillary comprises a positioning punch (6) provided with a tip-shaped distal end (61) intended for anchorage in a vertebra (VE), said positioning punch (6) being shaped so as to slidably guide the primary support (7) longitudinally by insertion of the positioning punch (6) inside the distal hole (73).

16. The implantation ancillary according to any one of claims 9 to 15, wherein the support means (94, 95) provided on the distal portion (91) of the implant-holder (9) comprise complementary pivot means (941, 951) shaped so as to cooperate with pivot means (23) of a rear portion (2) of the implant in order to enable a pivoting of said interspinous vertebral implant (1) around a transverse axis (AT).

17. The implantation ancillary according to any one of claims 9 to 16, wherein the implantation ancillary comprises an impactor (13) removably mounted on the proximal portion (92) of the implant-holder (9).
